(19) Europäisches Patentamt
European Patent Office
Office européen des brevets

(11) EP 0 653 202 B1

# (12) FASCICULE DE BREVET EUROPEEN

(45) Date de publication et mention de la délivrance du brevet:
**04.03.1998 Bulletin 1998/10**

(51) Int. Cl.[6]: **A61K 7/09**, C07C 323/41, C07C 323/12

(21) Numéro de dépôt: **94402397.7**

(22) Date de dépôt: **25.10.1994**

(54) **Composition cosmétique contenant en tant qu'agent réducteur un N-mercapto-alkyl alcanediamide ou l'un de ses sels cosmétiquement acceptable**

Kosmetische Zusammensetzung, enthaltend als Reduktionsmittel ein N-Mercaptoalkylalkandiamid oder eines seiner kosmetisch verträglichen Salze

Cosmetic composition containing an N-mercaptoalkyl alkanediamide or one of its cosmetically acceptable salts as a reducing agent

(84) Etats contractants désignés:
**AT BE CH DE ES FR GB IT LI NL SE**

(30) Priorité: **12.11.1993 FR 9313517**

(43) Date de publication de la demande:
**17.05.1995 Bulletin 1995/20**

(73) Titulaire: **L'OREAL**
**75008 Paris (FR)**

(72) Inventeur: **Malle, Gérard**
**F-77580 Villiers sur Morin (FR)**

(74) Mandataire:
**Tezier Herman, Béatrice**
**L'OREAL,**
**90, rue du Géneral Roguet**
**92583 Clichy Cédex (FR)**

(56) Documents cités:

| | |
|---|---|
| EP-A- 0 038 758 | EP-A- 0 161 769 |
| EP-A- 0 432 000 | EP-A- 0 465 342 |
| EP-A- 0 514 282 | FR-A- 2 669 329 |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.15.12/3.4

## Description

La présente invention a pour objet une composition cosmétique pour le premier temps d'une opération de déformation permanente des cheveux contenant en tant qu'agent réducteur un N-mercaptoalkyl alcanediamide ou l'un de ses sels cosmétiquement acceptables, et son utilisation dans un procédé de déformation permanente des cheveux. Elle a également pour objet de nouveaux disulfures de N-mercaptoalkyl alcanediamide ainsi que des nouveaux N-mercaptoalkyl alcanediamides.

La technique pour réaliser la déformation permanente des cheveux consiste, dans un premier temps, à réaliser l'ouverture des liaisons disulfures de la kératine (cystine) à l'aide d'une composition contenant un agent réducteur (étape de réduction), puis, après avoir de préférence rincé la chevelure, à reconstituer dans un second temps lesdites liaisons disulfures en appliquant, sur les cheveux sous tension, une composition oxydante, (étape d'oxydation, dite aussi de fixation) de façon à donner aux cheveux la forme recherchée. Cette technique permet indifféremment de réaliser soit l'ondulation des cheveux, soit leur défrisage ou leur décrêpage.

Les compositions pour réaliser le premier temps d'une opération de permanente se présentent généralement sous forme de lotions, de crèmes, de gels ou de poudres à diluer dans un support liquide et contiennent, en tant qu'agent réducteur, de préférence un thiol.

Parmi ces derniers, ceux couramment utilisés sont la cystéine et l'acide thioglycolique ainsi que ses esters, notamment le monothioglycolate de glycérol.

L'acide thioglycolique est particulièrement efficace pour réduire les liaisons disulfures de la kératine et peut être considéré à pH alcalin, notamment sous forme de thioglycolate d'ammonium, comme le composé de référence en permanente. Il présente cependant l'inconvénient de dégager une odeur désagréable. En vue d'y remédier, il est généralement fait usage d'un parfum permettant de masquer les odeurs.

La cystéine produit une odeur beaucoup plus faible que celle de l'acide thioglycolique mais le degré de frisure obtenu est très inférieur et loin d'être satisfaisant. De plus, la cystéine nécessite l'utilisation d'un pH très alcalin.

Le monothioglycolate de glycérol est également très malodorant. Il est par contre utilisé à un pH proche de la neutralité mais ses performances sont notablement inférieures à celles de l'acide thioglycolique.

Diverses études ont été conduites en vue de remédier aux inconvénients de ces agents réducteurs, et à cet effet il a été proposé l'emploi de nouveaux composés réducteurs. Ainsi, dans la demande de brevet européen EP-A-465 342, il a été décrit l'utilisation de dérivés d'acide N-(mercaptoalkyl) succinamique, ainsi d'ailleurs que les imides correspondantes.

La demanderesse a maintenant découvert que la mise en oeuvre des amides des dérivés d'acide N-(mercaptoalkyl)-succinamique, à molarité équivalente, permettait d'obtenir des rendements de frisure très supérieurs à ceux obtenus à l'aide des acides correspondants et à celui obtenu, selon l'état de la technique, à l'aide de l'acide thioglycolique.

Cette famille d'amides présente en effet des performances de frisure tout à fait exceptionnelles tout en ne possédant pas l'odeur soufrée propre aux thiols. Toutefois, il est bien connu que plus le rendement de frisure est élevé, plus les cheveux sont abîmés. Or, on a constaté de façon tout à fait surprenante qu'avec ces composés, l'amélioration de la frisure était obtenue sans détérioration de l'état des cheveux. Les agents réducteurs des compositions selon l'invention permettent par ailleurs d'obtenir une nervosité et une beauté de frisure supérieures à celles obtenues selon l'état de la technique.

L'invention a donc pour objet une composition cosmétique pour le premier temps d'une opération de déformation permanente des cheveux consistant à réduire les liaisons disulfures de la kératine, caractérisée par le fait qu'elle contient au moins un composé ayant la formule générale suivante :

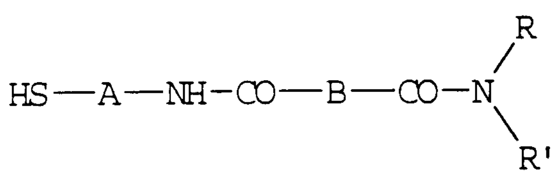

(I)

dans laquelle :

- A représente le radical divalent $-(CH_2)_n-$, n étant un nombre entier compris entre 2 et 5, ou le radical divalent $-(CH_2)_2-O-(CH_2)_2-$.

- B représente un radical choisi parmi le groupe comprenant :

(a) le radical divalent $-(CH_2)_m-$, m étant un nombre entier compris entre 1 et 7,

(b) le radical divalent

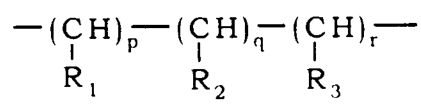

dans lequel l'un des groupes $R_1$, $R_2$, $R_3$ représente un radical choisi parmi le groupe comprenant les radicaux alkyle, linéaires ou ramifiés, ayant de 1 à 4 atomes de carbone, méthoxyméthyle, phényle, benzyle, cyclohexyle, cyclopentyle et les deux autres groupes sont un atome d'hydrogène, p, q et r étant O ou un nombre entier compris entre 1 et 4 et $1 \leq p + q + r \leq 4$,

(c) le radical divalent

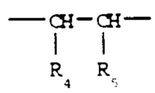

dans lequel $R_4$ et $R_5$ soit, identiques ou différents, représentent un radical alkyle, linéaire ou ramifié, ayant de 1 à 4 atomes de carbone, soit forment ensemble, avec les atomes de carbone adjacents, un cycle cyclohexane ou cyclohexène,

(d) le radical divalent

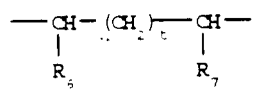

dans lequel l'un des groupes $R_6$ ou $R_7$ représente un radical $-NH_2$, et l'autre groupe représentant un atome d'hydrogène t étant égal à 0 ou 1, et

(e) le radical divalent

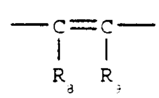

dans lequel $R_8$ et $R_9$, soit, identiques ou différents, représentent un atome d'hydrogène, un radical alkyle, linéaire ou ramifié, ayant de 1 à 4 atomes de carbone, soit forment ensemble, avec les atomes de carbone adjacents, un cycle benzénique.

- R et R', identiques ou différents, représentent :

  . soit un atome d'hydrogène, ou un radical choisi parmi le groupe comprenant les radicaux alkyle, linéaires ou ramifiés, ayant de 1 à 4 atomes de carbone, les radicaux hydroxyalkyle, linéaires ou ramifiés, ayant de 1 à 5 atomes de carbone,

  . soit R désigne un atome d'hydrogène et R' un radical aminoalkyle $-(CH_2)_v-NR_{10}R_{11}$ dans lequel v est un nombre entier compris entre 1 et 3 et $R_{10}$ et $R_{11}$, identiques ou différents, représentent un atome d'hydrogène ou un radical alkyle, linéaire ou ramifié, ayant de 1 à 3 atomes de carbone, $R_{10}$ et $R_{11}$ ne pouvant simultanément représenter un atome d'hydrogène,

et les sels organiques et minéraux desdits composés de formule (I).

Parmi les sels cosmétiquement acceptables des composés de formule (I), ceux particulièrement préférés sont les chlorhydrates, bromhydrates, citrates, oxalates et acétates.

A titre de préférence, le radical hydroxyalkyle ayant de 1 à 5 atomes de carbone est choisi parmi les radicaux hydroxy-2 éthyle, hydroxy-2 propyle, hydroxy-3 propyle, hydroxyméthyl-1 propyle, dihydroxyméthyl-1,1 propyle, tri-(hydroxyméthyl)-1,1,1, méthyle.

Parmi les composés préférés correspondant à la formule générale (I), on peut notamment citer :

. lorsque B représente le radical divalent $-CH_2-$ :

- le N-(2-mercapto-éthyl)-malonamide
- le N-(3-mercapto-propyl)-malonamide
- le N-(5-mercapto-pentyl)-malonamide
- le N-[2-(2-mercapto-éthoxy)-éthyl]-malonamide
- le N-(2-mercapto-éthyl)-N'-méthyl-malonamide
- le N-(2-mercapto-éthyl)-N',N'-diméthyl-malonamide
- le N-(2-mercapto-éthyl)-N'-éthyl-malonamide
- le N-(2-mercapto-éthyl)-N',N'-diéthyl-malonamide
- le N-(2-mercapto-éthyl)-N'-(2-hydroxy-éthyl)-malonamide
- le N-(2-mercapto-éthyl)-N',N'(2,2-dihydroxy-éthyl)-malonamide
- le N-(2-mercapto-éthyl)-N'-(3-diméthylamino-propyl)-malonamide
- le N-(2-mercapto-éthyl)-N'-(3-diéthylamino-propyl)-malonamide.

. lorsque B représente le radical divalent $-(CH_2)_2-$ :

- le N-(2-mercapto-éthyl)-succinamide
- le N-(3-mercapto-propyl)-succinamide
- le N-(5-mercapto-pentyl)-succinamide
- le N-[2-(2-mercapto-éthoxy)-éthyl]-succinamide
- le N-(2-mercapto-éthyl)-N'-méthyl-succinamide
- le N-(2-mercapto-éthyl)-N',N'-diméthyl-succinamide
- le N-(2-mercapto-éthyl)-N'-éthyl-succinamide
- le N-(2-mercapto-éthyl)-N',N'-diéthyl-succinamide
- le N-(2-mercapto-éthyl)-N'-(2-hydroxy-éthyl)-succinamide
- le N-(2-mercapto-éthyl)-N',N'-(2,2-dihydroxy-éthyl)-succinamide
- le N-(2-mercapto-éthyl)-N'-(3-diméthylamino-propyl)-succinamide
- le N-(2-mercapto-éthyl)-N'-(3-diéthylamino-propyl)-succinamide

. lorsque B représente le radical divalent $-(CH_2)_3-$ :

- le N-(2-mercapto-éthyl)-glutaramide
- le N-(3-mercapto-propyl)-glutaramide
- le N-(5-mercapto-pentyl)-glutaramide
- le N-[2-(2-mercapto-éthoxy)-éthyl]-glutaramide
- le N-(2-mercapto-éthyl)-N'-méthyl-glutaramide
- le N-(2-mercapto-éthyl)-N',N'-diméthyl-glutaramide
- le N-(2-mercapto-éthyl)-N'-éthyl-glutaramide
- le N-(2-mercapto-éthyl)-N',N'-diéthyl-glutaramide
- le N-(2-mercapto-éthyl)-N'-(2-hydroxy-éthyl)-glutaramide
- le N-(2-mercapto-éthyl)-N',N'-(2,2-dihydroxy-éthyl)-glutaramide
- le N-(2-mercapto-éthyl)-N'-(3-diméthylamino-propyl)-glutaramide

. lorsque B représente le radical divalent $-(CH_2)_4-$ :

- le N-(2-mercapto-éthyl)-adipamide
- le N-(2-mercapto-éthyl)-N'-méthyl-adipamide
- le N-(2-mercapto-éthyl)-N',N'-diméthyl-adipamide
- le N-(2-mercapto-éthyl)-N'-éthyl-adipamide

- le N-(2-mercapto-éthyl)-N'-(2-hydroxy-éthyl)-adipamide
- le N-(2-mercapto-éthyl)-N',N'-(2,2-dihydroxy-éthyl)-adipamide
- le N-(2-mercapto-éthyl)-N'-(3-diméthylamino-propyl)-adipamide.

. lorsque B représente le radical divalent

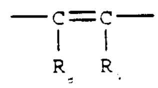

$R_8$ et $R_9$ ayant les mêmes significations que celles données ci-dessus :

- le N-(2-mercapto-éthyl)-fumaramide
- le N-(3-mercapto-propyl)-fumaramide
- le N-(5-mercapto-pentyl)-fumaramide
- le N-[2-(2-mercapto-éthoxy)-éthyl]-fumaramide
- le N-(2-mercapto-éthyl)-N'-méthyl-fumaramide
- le N-(2-mercapto-éthyl)-N',N'-diméthyl-fumaramide
- le N-(2-mercapto-éthyl)-N'-éthyl-fumaramide
- le N-(2-mercapto-éthyl)-N',N'-diéthyl-fumaramide
- le N-(2-mercapto-éthyl)-N'-(2-hydroxy-éthyl)-fumaramide
- le N-(2-mercapto-éthyl)-N',N'-(2,2-dihydroxy-éthyl)-fumaramide
- le N-(2-mercapto-éthyl)-N'-(3-diméthylamino-propyl)-fumaramide
- le N-(2-mercapto-éthyl)-téréphtalamide
- le N-(2-mercapto-éthyl)-N'-méthyl-téréphtalamide
- le N-(2-mercapto-éthyl)-N'-éthyl-téréphtalamide
- le N-(2-mercapto-éthyl)-N'-(2-hydroxy-éthyl)-téréphtalamide
- le N-(2-mercapto-éthyl)-N'-(3-diméthylamino-propyl)-téréphtalamide

. lorsque B représente le radical divalent $-(CH_2)_m-$, m étant un nombre entier compris entre 6 et 7 :

- l'acide octanedioïque 1-amide 5-[(2-mercapto-éthyl)-amide]
- l'acide nonanedioïque 1-amide 5-[(2-mercapto-éthyl)-amide]

. lorsque B représente le radical divalent

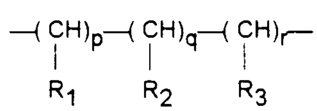

$R_1$, $R_2$, $R_3$, p, q, r ayant les mêmes significations que celles données ci-dessus :

- l'acide 3-méthyl-pentanedioïque 1-amide 5-[(2-mercapto-éthyl)-amide]
- l'acide 2-éthyl-hexanedioïque 1-amide 6-[(2-mercapto-éthyl)-amide]

. lorsque B représente le radical divalent

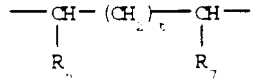

$R_6$, $R_7$, t ayant les mêmes significations que celles données ci-dessus :

- l'acide 4-amino-pentanedioïque 1-amide 5-[(2-mercapto-éthyl)-amide]

- l'acide 3-amino-butanedioïque 1-amide 5-[(2-mercapto-éthyl)-amide]

Dans les compositions selon l'invention, l'agent réducteur de formule générale (I) est généralement présent à une concentration comprise entre 0,5 et 30 % et de préférence entre 5 et 20 % en poids par rapport au poids total de la composition réductrice.

Le pH de la composition est de préférence compris entre 4 et 11 et plus particulièrement entre 6 et 10 et est obtenu à l'aide d'un agent alcalin tel que par exemple l'ammoniaque, la monoéthanolamine, la diéthanolamine, la triéthanolamine, la propanediamine-1,3, le carbamate d'ammonium, un carbonate ou bicarbonate alcalin ou d'ammonium, un carbonate organique tel que le carbonate de guanidine, un hydroxyde alcalin ou à l'aide d'un agent acidifiant tel que par exemple l'acide chlorhydrique, l'acide acétique, l'acide lactique, l'acide oxalique ou l'acide borique.

La composition réductrice peut également contenir en association un autre agent réducteur connu tel que par exemple l'acide thioglycolique, le monothioglycolate de glycérol ou de glycol, la cystéamine et ses dérivés acylés en $C_1$-$C_4$ tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide β-mercaptopropionique et ses dérivés, l'acide thiolactique et ses esters tel que le monothiolactate de glycérol, l'acide thiomalique, la panthétéine, le thioglycérol, les sulfites ou les bisulfites d'un métal alcalin ou alcalino-terreux, les N-(mercaptoalkyl)ω-hydroxyalkylamides décrits dans la demande de brevet EP-A-354.835 et les N-mono- ou N,N-dialkylmercapto 4-butyramides décrits dans la demande de brevet EP-A-368.763, les aminomercaptoalkylamides décrits dans la demande de brevet EP-A-432.000, les dérivés des acides N-(mercaptoalkyl) succinamiques ou des N-(mercaptoalkyl) succiminides décrits dans la demande de brevet EP-A-465.342, les alkylaminomercaptoalkylamides décrits dans la demande de brevet EP-A-514.282, le mélange de thioglycolate d'hydroxy-2-propyle et de thioglycolate d'hydroxy-2-méthyl-1 éthyle décrit dans la demande de brevet FR-A-2.679.448.

Selon un mode de réalisation préféré, la composition réductrice contient également un agent tensioactif de type non-ionique, anionique, cationique ou amphotère couramment utilisé dans les compositions réductrices de permanentes et, parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque la composition réductrice contient au moins un agent tensioactif, celui-ci est généralement présent à une concentration maximale de 30 % en poids, mais de préférence comprise entre 0,5 et 10 % en poids par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les brevet français FR-A-2.598.613 et FR-A-2.470.596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français FR-A-2.535.730, les polyorganosiloxanes à groupements aminoalkyles modifiés par des groupements alcoxycarbonyalkyles tels que ceux décrits dans le brevet US 4.749.732, des polyorganosiloxanes tels que le copolymère polydiméthylsiloxane-polyoxyalkyle du type Diméthicone Copolyol, un polydiméthylsiloxane à groupements terminaux stéaroxy (stéaroxydiméthicone), un copolymère polydiméthylsiloxane-dialkylammonium acétate ou un copolymère polydiméthyl-siloxane polyalkylbétaïne décrits dans GB-A-2.197.352, des polysiloxanes organo modifiés par des groupements mercapto ou mercaptoalkyles tels que ceux décrits dans le brevet français FR-A-1.530.369 et dans la demande de brevet européen EP-A-295.780, ainsi que des silanes tels que le stéaroxytriméthylsilane.

La composition réductrice peut également contenir d'autres ingrédients traitants tels que des polymères cationiques tels que ceux utilisés dans les compositions des brevets français FR-A-2.472.382 et FR-A-2.495.931, ou encore des polymères cationiques du type ionène tels que ceux utilisés dans les compositions du brevet luxembourgeois 83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration permettant de renforcer l'efficacité du réducteur tels que le mélange $SiO_2$/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monoéthyléther de propylèneglycol, le monométhyléther de dipropylèneglycol, le monoéthyléther de l'éthylèneglycol et le monoéthyléther du diéthylèneglycol, des alcanediols en $C_3$-$C_6$ tels que par exemple le propanediol-1,2 et le butanediol-1,2, l'imidazolidone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

La composition réductrice selon l'invention se présente essentiellement sous forme aqueuse notamment sous la

forme d'une lotion épaissie ou non, d'une crème ou d'un gel.

La composition réductrice selon l'invention peut être également du type exothermique, c'est-à-dire provoquant un certain échauffement lors de l'application sur les cheveux, ce qui apporte un agrément à la personne qui subit le premier temps de la permanente ou du défrisage.

Le véhicule des compositions selon l'invention est de préférence de l'eau ou une solution hydroalcoolique d'un alcool inférieur tel que l'éthanol, le propanol, l'isopropanol, le butanol ou d'un polyol tel que le glycérol, à une concentration maximale de 20 %. Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc...

On peut également utiliser des liquides ou des gels contenant des agents épaississants tels que des polymères ou des copolymères carboxyvinyliques qui "collent" les cheveux et les maintiennent dans la position lisse pendant le temps de pose.

Les compositions selon l'invention peuvent être également sous forme dite "auto-neutralisante" ou encore "auto-régulée" et dans ce cas, le composé de formule générale (I) est associé à au moins un disulfure soit connu pour son utilisation dans une composition réductrice pour permanente auto-neutralisante soit dérivant d'un composé de formule générale (I) ou de l'un de ses sels et correspondant à la formule générale (II) suivante :

$$—(—S—A—NH—CO—B—CO—NRR')_2 \qquad (II)$$

dans laquelle A, B, R et R', identiques ou différents de A, B, R et R' de la formule générale (I), ont les mêmes significations que celles données pour la formule générale (I).

Le disulfure peut également se présenter sous forme d'un sel cosmétiquement acceptable.

Parmi les disulfures connus, on peut notamment mentionner l'acide dithioglycolique, le dithioglycérol, la cystamine, la N, N'-diacétyl-cystamine, la cystine, la pantéthine, et les disulfures des N-(mercapto-alkyl)ω-hydroxyalkylamides décrits dans la demande de brevet européen EP-A-354.835, les disulfures des N-mono ou N,N-dialkylmercapto-4 butyramides décrits dans la demande de brevet EP-A-368.763, les disulfures des aminomercapto-alkylamides décrits dans la demande de brevet EP-A-432.000, les disulfures des dérivés des acides N-(mercaptoalkyl)-succinamiques ou des N-(mercaptoalkyl)-succinimides décrits dans la demande de brevet EP-A-465.342 et les disulfures des alkylaminomercaptoalkylamides décrits dans la demande de brevet EP-A-514.282.

Parmi les disulfures de formule générale (II), on peut citer :

. lorsque B désigne le radical divalent $-CH_2-$ :

- le disulfure du N-(2-mercapto-éthyl)-malonamide
- le disulfure du N-(3-mercapto-propyl)-malonamide
- le disulfure du N-(5-mercapto-pentyl)-malonamide
- le disulfure du N-[2-(2-mercapto-éthoxy)-éthyl]-malonamide
- le disulfure du N-(2-mercapto-éthyl)-N'-méthyl-malonamide
- le disulfure du N-(2-mercapto-éthyl)-N',N'-diméthyl-malonamide
- le disulfure du N-(2-mercapto-éthyl)-N'-éthyl-malonamide
- le disulfure du N-(2-mercapto-éthyl)-N',N'-diéthyl-malonamide
- le disulfure du N-(2-mercapto-éthyl)-N'-(2-hydroxy-éthyl)-malonamide
- le disulfure du N-(2-mercapto-éthyl)-N',N'-(2,2-dihydroxy-éthyl)-malonamide
- le disulfure du N-(2-mercapto-éthyl)-N'-(3-diméthylamino-propyl)-malonamide
- le disulfure du N-(2-mercapto-éthyl)-N'-(3-diéthylamino-propyl)-malonamide

. lorsque B désigne le radical divalent $-(CH_2)_2-$ :

- le disulfure du N-(2-mercapto-éthyl)-succinamide
- le disulfure du N-(3-mercapto-propyl)-succinamide
- le disulfure du N-(5-mercapto-pentyl)-succinamide
- le disulfure du N-[2-(2-mercapto-éthoxy)-éthyl]-succinamide
- le disulfure du N-(2-mercapto-éthyl)-N'-méthyl-succinamide
- le disulfure du N-(2-mercapto-éthyl)-N'-(2-hydroxy-éthyl)-succinamide
- le disulfure du N-(2-mercapto-éthyl)-N'-(3-diméthylamino-propyl)-succinamide

. lorsque B désigne le radical divalent $-(CH_2)_3-$ :

- le disulfure du N-(2-mercapto-éthyl)-glutaramide

. lorsque B désigne le radical divalent $-(CH_2)_4-$ :

- le disulfure du N-(2-mercapto-éthyl)-adipamide

Dans les compositions auto-neutralisantes, le disulfure est généralement présent en un rapport molaire de 0,5 à 2,5 et de préférence de 1 à 2 par rapport au composé de formule générale (I) ou de ses sels (voir brevet US 3.768.490).

La présente invention a en outre pour objet un procédé de déformation permanente des cheveux consistant, dans une première étape, à réduire les liaisons disulfures de la kératine par application, pendant environ 5 à 60 min, d'une composition réductrice telle que définie ci-dessus puis, dans une seconde étape, à reformer lesdites liaisons par application d'une composition oxydante ou éventuellement en laissant agir l'oxygène de l'air.

La présente invention a également pour objet un procédé d'ondulation des cheveux dans lequel on applique une composition réductrice telle que définie ci-dessus sur des cheveux mouillés préalablement enroulés sur des rouleaux ayant de 4 à 20 mm de diamètre, la composition pouvant éventuellement être appliquée au fur et à mesure de l'enroulage des cheveux : on laisse ensuite agir la composition réductrice pendant un temps de 5 à 60 minutes, de préférence de 5 à 30 minutes, puis on rince abondamment après quoi on applique, sur les cheveux enroulés, une composition oxydante permettant de reformer les liaisons disulfures de la kératine pendant un temps de pose de 2 à 10 minutes. Après avoir enlevé les rouleaux, on rince abondamment la chevelure.

La composition d'oxydation ou oxydante est du type couramment utilisé et contient comme agent oxydant de l'eau oxygénée, un bromate alcalin, un persel, un polythionate ou un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 % en poids par rapport au poids total de la composition oxydante. Le pH de la composition oxydante est généralement compris entre 2 et 10. Cette oxydation peut être effectuée immédiatement ou être différée.

La présente invention a également pour objet un procédé de défrisage ou de décrêpage des cheveux dans lequel on applique sur les cheveux une composition réductrice selon l'invention puis l'on soumet les cheveux à une déformation mécanique permettant de les fixer dans leur nouvelle forme, par une opération de lissage des cheveux avec un peigne à larges dents, avec le dos d'un peigne ou à la main. Après un temps de pose de 5 à 60 minutes, en particulier de 5 à 30 minutes, on procède alors à un nouveau lissage puis on rince soigneusement et on applique une composition oxydante ou fixatrice telle que définie ci-dessus, que l'on laisse agir pendant environ 2 à 10 minutes puis on rince abondamment les cheveux.

L'invention a également pour objet le procédé de préparation des N-mercaptoalkyl alcanediamides de formule (I). Ce procédé consiste à faire réagir un aminothiol (1) sur un monoester de diacide (2) de façon à obtenir un N-mercaptoalkylamide ester (3) qui par traitement avec une amine primaire ou secondaire ou par l'ammoniaque conduit aux N-mercaptoalkyl alcanediamides de formule (I) selon le schéma réactionnel suivant :

$$\underset{(1)}{HS{-}A{-}NH_2} \quad + \quad \underset{(2)}{HOOC{-}B{-}COOR_{12}} \longrightarrow$$

$$\underset{(3)}{HS{-}A{-}NH{-}CO{-}B{-}COOR_{12}} \xrightarrow{HNRR'}$$

$$HS{-}A{-}NH{-}CO{-}B{-}CO{-}NRR' \qquad (I)$$

La réaction entre l'aminothiol (1) et le monoester de diacide (2) est conduite sous atmosphère inerte dans un solvant inerte tel que par exemple le dichlorométhane, le dichloro-1,2 éthane, le trichloro-1,1,1 éthane, le chloroforme, l'acétonitrile, le toluène, le dioxanne, le tétrahydrofuranne, le diméthoxy-1,2 éthane, le cyclohexane ou un mélange de ces solvants, à température ambiante en présence d'agents favorisant le couplage tels que le dicyclohexylcarbodiimide. Le radical $R_{12}$ est un reste alkyle ayant de préférence de 1 à 4 atomes de carbone, avantageusement méthyle ou éthyle. A, B, R, R' ont les mêmes significations que celles données pour la formule générale. On met de préférence en réaction l'aminothiol (1) sous la forme de son chlorhydrate et dans ce cas on ajoute dans le milieu réactionnel un équivalent d'une base telle que par exemple la triéthylamine.

La réaction d'amidification du composé (3) par une amine primaire ou secondaire est conduite soit sans solvant soit en présence d'un solvant tel qu'un alcool inférieur linéaire ou ramifié, comme par exemple, le méthanol, à une température comprise entre la température ambiante et le point d'ébullition de l'alcool utilisé. La réaction d'amidification du composé (3) par l'ammoniaque est réalisée soit avec de l'ammoniac gazeux dans un solvant inerte, soit avantageusement à l'aide d'une solution aqueuse d'ammoniaque à température ambiante.

Les disulfures des composés de formule (I) sont obtenus de façon classique, par oxydation des composés de formule générale (I), soit à l'air, soit à l'aide d'oxydants connus comme par exemple l'eau oxygénée en présence éventuellement de sels métalliques tels que par exemple les sels ferreux.

La présente invention a également pour objet les nouveaux disulfures de formule (II) suivante :

$$-(-S-A-NH-CO-B-CO-NRR')_2 \qquad (II)$$

dans laquelle A, B, R et R' ont les même significations que celles données ci-dessus, et les sels organiques et minéraux desdits composés de formule (II ).

La présente invention a également pour objet les nouveaux N-mercaptoalkyl alcanediamides de formule (III) suivante :

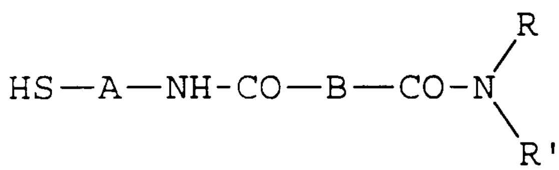

(III)

dans laquelle :

- A représente le radical divalent $-(CH_2)_n-$, n étant un nombre entier compris entre 2 et 5, ou le radical divalent $-(CH_2)_2-O-(CH_2)_2-$.
- B représente un radical choisi parmi le groupe comprenant :

  (a) le radical divalent $-(CH_2)_m-$, m étant un nombre entier compris entre 1 et 7,
  (b) le radical divalent

  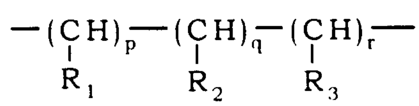

  dans lequel l'un des groupes $R_1$, $R_2$, $R_3$ représente un radical choisi parmi le groupe comprenant les radicaux alkyle, linéaires ou ramifiés, ayant de 1 à 4 atomes de carbone, méthoxyméthyle, phényle, benzyle, cyclohexyle, cyclopentyle et les deux autres groupes sont un atome d'hydrogène, p, q et r étant 0 ou un nombre entier compris entre 1 et 4 et $1 \leq p + q + r \leq 4$ ,
  (c) le radical divalent

  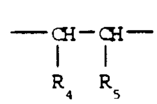

  dans lequel $R_4$ et $R_5$ soit, identiques ou différents, représentent un radical alkyle, linéaire ou ramifié, ayant de 1 à 4 atomes de carbone, soit forment ensemble, avec les atomes de carbone adjacents, un cycle cyclohexane ou cyclohexène,
  (d) le radical divalent

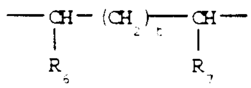

dans lequel l'un des groupes $R_6$ ou $R_7$ représente un radical $-NH_2$, et l'autre groupe représentant un atome d'hydrogène t étant égal à 0 ou 1, et

(e) le radical divalent

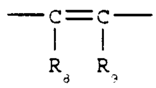

dans lequel $R_8$ et $R_9$, soit, identiques ou différents, représentent un atome d'hydrogène, un radical alkyle, linéaire ou ramifié, ayant de 1 à 4 atomes de carbone, soit forment ensemble, avec les atomes de carbone adjacents, un cycle benzénique.

- R et R', identiques ou différents, représentent :

  . soit un atome d'hydrogène, ou un radical choisi parmi le groupe comprenant les radicaux alkyle, linéaires ou ramifiés, ayant de 1 à 4 atomes de carbone, les radicaux hydroxyalkyle, linéaires ou ramifiés, ayant de 1 à 5 atomes de carbone,

  . soit R désigne un atome d'hydrogène et R' un radical aminoalkyle $-(CH_2)_v-NR_{10}R_{11}$ dans lequel v est un nombre entier compris entre 1 et 3 et $R_{10}$ et $R_{11}$, identiques ou différents, représentent un atome d'hydrogène ou un radical alkyle, linéaire ou ramifié, ayant de 1 à 3 atomes de carbone, $R_{10}$ et $R_{11}$ ne pouvant simultanément représenter un atome d'hydrogène,

et les sels organiques et minéraux desdits composés de formule (III),
à l'exclusion des composés dans lesquels :
soit

(i) A représente le radical divalent $-(CH_2)_2-$, et B représente le radical $-(CH_2)_m-$, m étant un nombre entier compris entre 2 et 7
ou le radical

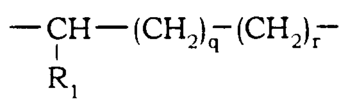

dans lequel $R_1$ représente un radical choisi parmi le groupe comprenant les radicaux alkyle, linéaires ou ramifiés, ayant de 1 à 4 atomes de carbone, phényle, benzyle, cyclohexyle, cyclopentyle, $1 < 1 + q + r \leq 4$, et R et R', identiques ou différents, représentent :

  . soit un atome d'hydrogène, ou un radical choisi parmi le groupe comprenant les radicaux alkyle, linéaires ou ramifiés, ayant de 1 à 4 atomes de carbone,

  . soit R désigne un atome d'hydrogène et R' un radical aminoalkyle $-(CH_2)_v-NR_{10}R_{11}$ dans lequel v est un nombre entier compris entre 1 et 3 et $R_{10}$ et $R_{11}$, identiques ou différents, représentent un atome d'hydrogène ou un radical alkyle, linéaire ou ramifié, ayant de 1 à 3 atomes de carbone, $R_{10}$ et $R_{11}$ ne pouvant simultanément représenter un atome d'hydrogène,

soit
(ii) A représente le radical divalent $-(CH_2)_2-$ et B représente le radical $-CH_2-$, ou le radical

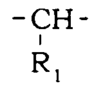

dans lequel $R_1$ représente un radical choisi parmi le groupe comprenant les radicaux alkyle, linéaires ou ramifiés, ayant de 1 à 4 atomes de carbone, méthoxyméthyle, phényle, benzyle,
et R et R', identiques ou différents, représentent :

- soit un atome d'hydrogène, ou un radical choisi parmi le groupe comprenant les radicaux alkyle, linéaires ou ramifiés, ayant de 1 à 4 atomes de carbone,
- soit R désigne un atome d'hydrogène et R' un radical aminoalkyle $-(CH_2)_v-NR_{10}R_{11}$ dans lequel v est un nombre entier compris entre 1 et 3 et $R_{10}$ et $R_{11}$, identiques ou différents, représentent un radical alkyle, linéaire ou ramifié, ayant de 1 à 3 atomes de carbone,

On va maintenant donner à titre d'illustration et sans aucun caractère limitatif plusieurs exemples de préparation de composés selon l'invention ainsi que des exemples de compositions réductrices et leur utilisation dans un procédé de déformation permanente des cheveux.

EXEMPLE 1: Préparation du N-(2-mercapto-éthyl)-succinamide

a) Ester méthylique de l'acide N-(2-mercapto-éthyl)-succinamique

A une suspension de 4,2 g (37 mmoles) de chlorhydrate de cystéamine dans 100 $cm^3$ de dichlorométhane, agitée sour argon à 0° C, on ajoute 5,2 $cm^3$ de triéthylamine puis 15 minutes plus tard 5,1 g d'hydroxy-1 benzotriazole et en 15 minutes une solution de 7,63 g de dicyclohexylcarbodiimide dans environ 30 $cm^3$ de dichlorométhane. On ajoute alors 5,15 g (38 mmoles) de monométhyl succinate et maintient l'agitation 12 heures à température ambiante.

On filtre le milieu réactionnel et extrait le filtrat successivement 3 fois par 50 $cm^3$ de solution aqueuse saturée de bicarbonate de sodium puis 3 fois par 50 $cm^3$ de solution aqueuse de bisulfate de potassium à 5 %. La phase organique est séchée sur sulfate de sodium puis évaporée à sec sous pression réduite. L'huile obtenue est purifiée par chromatographie sur gel de silice éluée à l'acétate d'éthyle. Après évaporation à sec et séchage sous vide à température ambiante, on obtient 5,7 g d'ester méthylique de l'acide N-(2-mercapto-éthyl) succinamique sous la forme d'une huile incolore.

Le spectre RMN $^1H$ 200 MHz ($CDCl_3$ + TMS) et dosage de thiol par iodométrie sont conformes à la structure attendue.

b) N-(2-mercapto-éthyl)-succinamide

Une solution de 5,7 g (30 mmoles) d'ester méthylique de l'acide N-(2-mercapto-éthyl)-succinamique, obtenu à l'exemple 1a), dans 50 $cm^3$ de solution aqueuse d'ammoniaque à 20 % est agitée 4 heures à température ambiante sous atmosphère d'argon.

Après évaporation à sec sous pression réduite, le solide obtenu est purifié par recristallisation dans l'éthanol. Après essorage et séchage sous vide à 50° C, on obtient 3,8 g de N-(2-mercapto-éthyl)-succinamide sous la forme d'un solide blanc de point de fusion 173° C.

Le spectre RMN $^1H$ 200 MHz (DMSO d6 + TMS) est conforme à la structure attendue. Le dosage de thiol est également conforme : trouvé : 5,55 méq/g ; calculé : 5,67 méq/g.

| ANALYSE ELEMENTAIRE : $C_6 H_{12} N_2 O_2 S$ | | | | | |
|---|---|---|---|---|---|
| | C % | H % | N % | O % | S % |
| Calculé | 40,89 | 6,86 | 15,90 | 18,16 | 18,19 |
| Trouvé | 40,75 | 6,85 | 16,25 | 18,80 | 18,11 |

EXEMPLE 2 : Préparation du N-(2-mercapto-éthyl)-N'-(2-hydroxy-éthyl)-succinamide

Une solution de 4,4 g (23 mmoles) d'ester méthylique de l'acide N-(2-mercapto-éthyl)-succinamique, obtenu à l'exemple 1a), dans 20 $cm^3$ de méthanol et 1,68 g (27 mmoles) d'éthanolamine est agitée 5 heures à 60° C sous atmosphère d'argon.

Après évaporation à sec sous pression réduite et recristallisation dans l'isopropanol, on obtient 2,8 g de N-(2-mercapto-éthyl)-N'-(2-hydroxy-éthyl)-succinamide sous la forme d'un solide blanc, de point de fusion : 140° C.

Le spectre RMN $^1H$ 200 MHz (DMSO d6 + TMS) est conforme à la structure attendue. Le dosage de thiol est également conforme : trouvé : 4,34 méq/g ; calculé : 4,54 méq/g.

| ANALYSE ELEMENTAIRE : $C_8 H_{16} N_2 O_3 S$ | | | | | |
|---|---|---|---|---|---|
| | C % | H % | N % | O % | S % |
| Calculé | 43,62 | 7,32 | 12,72 | 21,79 | 14,56 |
| Trouvé | 43,57 | 7,29 | 12,67 | 21,85 | 14,45 |

EXEMPLE 3 : Préparation du N-(2-mercapto-éthyl)-malonamide

a) Ester méthylique de l'acide N-(2-mercapto-éthyl)-malonamique

A une suspension de 75 g (0,66 mole) de chlorhydrate de cystéamine dans 700 $cm^3$ de dichlorométhane, agitée sous argon à 0° C, on ajoute 89 g d'hydroxy-1 benzotriazole puis, en environ 30 minutes, une solution de 136 g de dicyclohexylcarbodiimide dans 300 $cm^3$ de dichlorométhane. On ajoute ensuite 102 g (0,65 mole) de monométhyl malonate de potassium et maintient l'agitation 30 minutes à 0° C puis 4 heures à température ambiante.

Le milieu réactionnel est filtré. Le filtrat est extrait successivement avec une solution aqueuse de bicarbonate de sodium à 5 %, une solution aqueuse de bisulfate de potassium à 5 % et une fois à l'eau. La phase organique est séchée sur sulfate de sodium et évaporée à sec sous pression réduite. L'huile brute obtenue est purifiée par chromatographie sur gel de silice dans le mélange éluant dichlorométhane/méthanol 98/2. On obtient après évaporation à sec et séchage sous vide 53 g d'ester méthylique de l'acide N-(2-mercapto-éthyl)-malonamique sous la forme d'une huile incolore.

Le spectre RMN $^1H$ 200 MHz ($CDCl_3$ + TMS) et le dosage de thiol sont conformes à la structure attendue.

b) N-(2-mercapto-éthyl)-malonamide

A 220 $cm^3$ de solution aqueuse d'ammoniaque à 20 %, on ajoute, sous agitation 31 g d'ester méthylique de l'acide N-(2-mercapto-éthyl)-malonamique, obtenu à l'exemple 3a), en maintenant la température inférieure ou égale à 30° C. L'agitation est maintenue 1 heure à température ambiante. Un léger trouble est éliminé par filtration sur verre fritté. Le filtrat est évaporé à sec sous pression réduite. Le solide obtenu est recristallisé dans l'acétate d'éthyle. Après essorage et séchage sous vide à 50° C, on obtient 27 g de N-(2-mercapto-éthyl)-malonamide sous la forme d'un solide blanc de point de fusion : 100° C.

Le spectre RMN $^1H$ 200 MHz (DMSO d6 + TMS) est conforme à la structure attendue. Le dosage de thiol est également conforme : trouvé : 6,12 méq/g ; calculé : 6,17 méq/g.

| ANALYSE ELEMENTAIRE : $C_5 H_{10} N_2 O_2 S$ | | | | | |
|---|---|---|---|---|---|
| | C % | H % | N % | O % | S % |
| Calculé | 37,02 | 6,21 | 17,27 | 19,73 | 19,77 |
| Trouvé | 37,10 | 6,22 | 17,01 | 19,87 | 19,52 |

EXEMPLE 4 : Préparation du N-(2-mercapto-éthyl)-glutaramide

a) Ester méthylique de l'acide N-(2-mercapto-éthyl)-glutaramique

On procède de la même façon qu'à l'exemple 1a) en mettant en oeuvre :

. 6,2 g (54 mmoles) de chlorhydrate de cystéamine
. 7,7 $cm^3$ de triéthanolamine
. 7,3 g d'hydroxy-1 benzotriazole
. 11,3 g de dicyclohexylcarbodiimide
. 8,4 g (57 mmoles) de mono-méthyl glutarate.

On obtient 9,1 g d'ester méthylique de l'acide N-(2-mercapto-éthyl)-glutaramique sous la forme d'un solide blanc. Le spectre RMN $^1H$ 200 MHz ($CDCl_3$ + TMS) et le dosage de thiol sont conformes à la structure attendue.

b) N-(2-mercapto-éthyl)-glutaramide

Une solution de 9,1 g (44 mmoles) d'ester méthylique de l'acide N-(2-mercapto-éthyl)-glutaramique, obtenu à l'exemple 4a), dans 100 cm3 de solution aqueuse d'ammoniaque à 20 % est agitée 4 heures à température ambiante sous atmosphère d'argon. Après évaporation à sec et recristallisation dans l'isopropanol, on obtient 7 g de N-(2-mercapto-éthyl)-glutaramide sous la forme d'un solide blanc de point de fusion : 127° C.

Le spectre RMN $^1H$ 200 MHz (DMSO d6 + TMS) est conforme à la structure attendue. Le dosage de thiol est également conforme : trouvé : 5,10 méq/g ; calculé : 5,26 méq/g.

| ANALYSE ELEMENTAIRE : $C_7 H_{14} N_2 O_2 S$ | | | | | |
|---|---|---|---|---|---|
| | C % | H % | N % | O % | S % |
| Calculé | 44,20 | 7,42 | 14,72 | 16,82 | 16,85 |
| Trouvé | 43,70 | 7,51 | 14,66 | 17,39 | 16,74 |

EXEMPLE 5 : Préparation du disulfure du N-(2-mercapto-éthyl)-succinamide

20 g (0,113 mole) de N-(2-mercapto-éthyl)-succinamide, obtenu à l'exemple 1b), sont dissous dans 1 litre de méthanol. A la solution obtenue on ajoute, goutte-à-goutte, à température ambiante 6 $cm^3$ d'eau oxygénée à 30 % puis 50 mg de sulfate ferreux. Le disulfure précipite au fur et à mesure de sa formation. Après une nuit d'agitation, on refroidit à +5° C et essore sur verre fritté n° 3. Le disulfure brut est purifié par recristallisation dans un mélange éthanol/eau. On obtient ainsi après essorage et séchage sous vide à 40-50° C, 12 g de disulfure du N-(2-mercapto-éthyl)-succinamide sous la forme d'un solide blanc de point de fusion 218° C.

Les spectres RMN $^1H$ 200 MHz (DMSO + TMS) et $^{13}C$ 50 MHz (DMSO + TMS) sont conformes à la structure attendue.

| ANALYSE ELEMENTAIRE : $C_{12} H_{22} N_4 O_4 S_2$ | | | | | |
|---|---|---|---|---|---|
| | C % | H % | N % | O % | S % |
| Calculé | 41,13 | 6,33 | 15,99 | 18,26 | 18,30 |
| Trouvé | 40,90 | 6,19 | 15,76 | 18,61 | 18,14 |

EXEMPLE 6:

On prépare selon l'invention une composition réductrice de déformation permanente des cheveux en procédant au mélange des ingrédients suivants :

| | |
|---|---|
| - Composé de l'exemple 1 | 10 g |
| - N-laurylamide propyl bétaïne vendu sous la dénomination "Softazoline LPB" par la Société KAWAKEN Fine Chemicals | 0,35 g |
| - Monoéthanolamine qs | pH 8,4 |
| - Parfum qs | |
| - Eau déminéralisée qsp | 100 g |

Cette composition est appliquée sur des cheveux mouillés préalablement enroulés sur des rouleaux de mise en plis. Après avoir laissé agir la composition pendant environ 15 min., on rince abondamment à l'eau puis on applique la composition oxydante suivante :

| | |
|---|---|
| - Eau oxygénée qsp | 8 volumes |
| - Stabilisants qs | |
| - Oxyde de lauryl diméthyl amine | 0,7 g |
| - Parfum | |
| - Acide lactique qsp | pH 3.0 |
| - Eau déminéralisée qsp | 100 g |

On laisse agir la composition oxydante pendant environ 10 minutes, puis on enlève les rouleaux et rince abondamment la chevelure à l'eau.

Après séchage sous casque, les cheveux présentent de belles boucles.

EXEMPLE 7 :

Selon le même mode de réalisation qu'à l'exemple 1, on a procédé à une déformation permanente des cheveux à l'aide des compositions réductrices et oxydantes suivantes :

| *A - Composition réductrice* | |
|---|---|
| - Composé de l'exemple 1 | 8,5 g |
| - Cocoamidopropylbétaïne | 1 g |
| - Carbonate d'ammonium | 2 g |
| - Ammoniaque qs | pH 9 |
| - Eau déminéralisée qs | 100 g |

| *B - Composition oxydante* | |
|---|---|
| - Bromate de sodium | 8 g |
| - Triéthanolamine qsp | pH 8.0 |
| - Phosphate monosodique hydraté (12 $H_2O$) | 0,3 g |
| - Phosphate trisodique hydraté (2 $H_2O$) | 0,5 g |
| - Cocoylamidopropyl bétaïne avec monoglycéride de coprah vendu sous la dénomination de "TEGOBETAINE HS" par la Société GOLDSCHMIDT | 1 g |
| - Eau déminéralisée qsp | 100 g |

EXEMPLE 8 :

| *A - Composition réductrice* | |
|---|---|
| - Composé de l'exemple 1 | 9,5 g |
| - Chlorure de cétyltriméthylammonium | 0,5 g |
| - Glycérol | 2 g |
| - Triéthanolamine qs | pH 7,9 |
| - Eau déminéralisée qsp | 100 g |

| *B - Composition oxydante* | |
|---|---|
| - Eau oxygénée à 200 volumes | 4,8 g |
| - Sulfate d'hydroxy-8 quinoléine | 0,01 g |
| - Phénacétine | 0,05 g |
| - Acide citrique qsp | pH 3,0 |
| - Parfum | |
| - Eau déminéralisée qsp | 100 g |

EXEMPLE 9 :

On applique sur cheveux européens enroulés sur bigoudis de 9 mm de diamètre la composition réductrice suivante :

| | |
|---|---|
| - Composé de l'exemple 2 | 16 g |
| - Ammoniaque qsp | pH 9 |
| - N-laurypropyl bétaïne | 1 g |
| - Parfum qs | |
| - Eau déminéralisée qsp | 100 g |

On laisse poser à température ordinaire pendant 20 minutes. On rince puis on applique la composition oxydante de l'exemple 6.

Après 5 minutes de pose, on rince et on déroule.

Après séchage, les cheveux présentent de belles boucles et de bonnes propriétés cosmétiques.

EXEMPLE 10:

On applique sur cheveux européens enroulés sur bigoudis de 9 mm de diamètre la composition réductrice suivante :

| | |
|---|---|
| - Composé de l'exemple 3 | 10 g |
| -Monoéthanolamine qsp | pH 9,2 |
| - Cocoamidopropylbétaïne | 1 g |
| - Parfum qs | |
| - Eau déminéralisée qsp | 100 g |

On laisse poser à température ordinaire pendant 20 minutes. On rince puis on applique la composition oxydante de l'exemple 6.

Après 5 minutes de pose, on rince et on déroule.

Après séchage, les cheveux présentent de belles boucles et de bonnes propriétés cosmétiques.

EXEMPLE 11 :

On applique sur cheveux européens enroulés sur bigoudis de 9 mm de diamètre la composition réductrice suivante :

| | |
|---|---|
| - Composé de l'exemple 2 | 10 g |
| - Carbonate d'ammonium | 2 g |
| - Monoéthanolamine qsp | pH 9 |
| - Cocoamidopropylbétaïne | 1 g |
| - Parfum qs | |
| - Eau déminéralisée qsp | 100 g |

On laisse poser à température ordinaire pendant 20 minutes. On rince puis on applique la composition oxydante de l'exemple 6.

Après 5 minutes de pose, on rince et on déroule.

Après séchage, les cheveux présentent de belles boucles et de bonnes propriétés cosmétiques.

EXEMPLE 12 :

On applique sur cheveux européens enroulés sur bigoudis de 9 mm de diamètre la composition réductrice suivante :

| | |
|---|---|
| - Composé de l'exemple 3 | 10 g |
| - Composé de l'exemple 5 | 3 g |
| - Ammoniaque | 2 g |
| - Carbonate d'ammonium | 1 g |
| - Monoéthanolamine qsp | pH 9,2 |
| - Chlorure de cétyltriméthylammonium | 1 g |
| - Parfum qs | |
| - Eau déminéralisée qsp | 100 g |

On laisse poser à température ordinaire pendant 20 minutes. On rince puis on applique la composition oxydante de l'exemple 6.

Après 5 minutes de pose, on rince et on déroule.

Après séchage, les cheveux présentent de belles boucles et de bonnes propriétés cosmétiques.

EXEMPLE 13 :

On applique sur cheveux européens enroulés sur bigoudis de 9 mm de diamètre la composition réductrice suivante :

| | |
|---|---|
| - Composé de l'exemple 2 | 12 g |
| - Acide acétique | 0,2 g |
| - Monéthanolamine qsp | pH 7,3 |
| - Cocoamidopropylbétaïne | 1 g |
| - Parfum qs | |
| - Eau déminéralisée qsp | 100 g |

On laisse poser à température ordinaire pendant 20 minutes. On rince puis on applique la composition oxydante de l'exemple 6.

Après 5 minutes de pose, on rince et on déroule.

Après séchage, les cheveux présentent de belles boucles et de bonnes propriétés cosmétiques.

## Revendications

1. Composition cosmétique pour le premier temps d'une opération de déformation permanente des cheveux consistant à réduire les liaisons disulfures de la kératine, caractérisée par le fait qu'elle contient au moins un composé ayant la formule générale suivante :

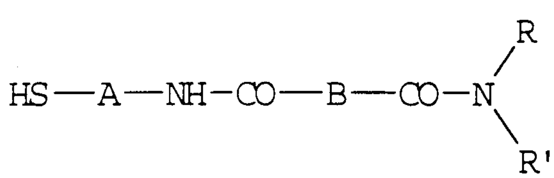

(I)

dans laquelle :

- A représente le radical divalent $-(CH_2)_n-$, n étant un nombre entier compris entre 2 et 5, ou le radical divalent $-(CH_2)_2-O-(CH_2)_2-$.
- B représente un radical choisi parmi le groupe comprenant :

    (a) le radical divalent $-(CH_2)_m-$, m étant un nombre entier compris entre 1 et 7,
    (b) le radical divalent

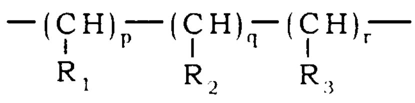

dans lequel l'un des groupes $R_1$, $R_2$, $R_3$ représente un radical choisi parmi le groupe comprenant les radicaux alkyle, linéaires ou ramifiés, ayant de 1 à 4 atomes de carbone, méthoxyméthyle, phényle, benzyle, cyclohexyle, cyclopentyle et les deux autres groupes sont un atome d'hydrogène, p, q et r étant O ou un nombre entier compris entre 1 et 4 et $1 \leq p + q + r \leq 4$,
(c) le radical divalent

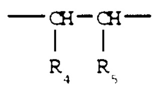

dans lequel $R_4$ et $R_5$ soit, identiques ou différents, représentent un radical alkyle, linéaire ou ramifié, ayant de 1 à 4 atomes de carbone, soit forment ensemble, avec les atomes de carbone adjacents, un cycle cyclohexane ou cyclohexène,
(d) le radical divalent

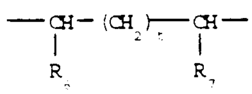

dans lequel l'un des groupes $R_6$ ou $R_7$ représente un radical $-NH_2$, et l'autre groupe représentant un atome d'hydrogène ; t étant égal à 0 ou 1, et
(e) le radical divalent

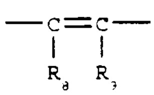

dans lequel $R_8$ et $R_9$, soit, identiques ou différents, représentent un atome d'hydrogène, un radical alkyle, linéaire ou ramifié, ayant de 1 à 4 atomes de carbone, soit forment ensemble, avec les atomes de carbone adjacents, un cycle benzénique.

- R et R', identiques ou différents, représentent :

    . soit un atome d'hydrogène, ou un radical choisi parmi le groupe comprenant les radicaux alkyle, linéaires ou ramifiés, ayant de 1 à 4 atomes de carbone, les radicaux hydroxyalkyle, linéaires ou ramifiés, ayant de 1 à 5 atomes de carbone,
    . soit R désigne un atome d'hydrogène et R' un radical aminoalkyle $-(CH_2)_v-NR_{10}R_{11}$ dans lequel v est un nombre entier compris entre 1 et 3 et $R_{10}$ et $R_{11}$, identiques ou différents, représentent un atome d'hydrogène ou un radical alkyle, linéaire ou ramifié, ayant de 1 à 3 atomes de carbone, $R_{10}$ et $R_{11}$ ne pouvant simultanément représenter un atome d'hydrogène,

et les sels organiques et minéraux desdits composés de formule (I).

2. Composition selon la revendication 1, caractérisée par le fait que les sels des composés de formule (I) sont choisis par les chlorhydrates, bromhydrates, citrates, oxalates et acétates.

3. Composition selon les revendications 1 ou 2, caractérisée par le fait que lorsque dans la formule générale (I), B représente le radical divalent $-CH_2-$, les composés sont choisis parmi :

- le N-(2-mercapto-éthyl)-malonamide
- le N-(3-mercapto-propyl)-malonamide
- le N-(5-mercapto-pentyl)-malonamide
- le N-[2-(2-mercapto-éthoxy)-éthyl]-malonamide
- le N-(2-mercapto-éthyl)-N'-méthyl-malonamide
- le N-(2-mercapto-éthyl)-N',N'-diméthyl-malonamide
- le N-(2-mercapto-éthyl)-N'-éthyl-malonamide
- le N-(2-mercapto-éthyl)-N',N'-diéthyl-malonamide
- le N-(2-mercapto-éthyl)-N'-(2-hydroxy-éthyl)-malonamide
- le N-(2-mercapto-éthyl)-N',N'-(2,2-dihydroxy-éthyl)-malonamide
- le N-(2-mercapto-éthyl)-N'-(3-diméthylamino-propyl)-malonamide
- le N-(2-mercapto-éthyl)-N'-(3-diéthylamino-propyl)-malonamide

4. Composition selon les revendications 1 ou 2, caractérisée par le fait que lorsque dans la formule générale (I) B représente le radical divalent $-(CH_2)_2-$, les composés sont choisis parmi :

- le N-(2-mercapto-éthyl)-succinamide
- le N-(3-mercapto-propyl)-succinamide
- le N-(5-mercapto-pentyl)-succinamide
- le N-[2-(2-mercapto-éthoxy)-éthyl]-succinamide
- le N-(2-mercapto-éthyl)-N'-méthyl-succinamide
- le N-(2-mercapto-éthyl)-N',N'-diméthyl-succinamide
- le N-(2-mercapto-éthyl)-N'-éthyl-succinamide
- le N-(2-mercapto-éthyl)-N',N'-diéthyl-succinamide
- le N-(2-mercapto-éthyl)-N'-(2-hydroxy-éthyl)-succinamide
- le N-(2-mercapto-éthyl)-N',N'-(2,2-dihydroxy-éthyl)-succinamide
- le N-(2-mercapto-éthyl)-N'-(3-diméthylamino-propyl)-succinamide
- le N-(2-mercapto-éthyl)-N'-(3-diéthylamino-propyl)-succinamide

5. Composition selon les revendications 1 ou 2, caractérisée par le fait que lorsque dans la formule générale (I) B représente le radical divalent $-(CH_2)_3-$, les composés sont choisis parmi :

- le N-(2-mercapto-éthyl)-glutaramide
- le N-(3-mercapto-propyl)-glutaramide
- le N-(5-mercapto-pentyl)-glutaramide
- le N-[2-(2-mercapto-éthoxy)-éthyl]-glutaramide
- le N-(2-mercapto-éthyl)-N'-méthyl-glutaramide
- le N-(2-mercapto-éthyl)-N',N'-diméthyl-glutaramide
- le N-(2-mercapto-éthyl)-N'-éthyl-glutaramide
- le N-(2-mercapto-éthyl)-N',N'-diéthyl-glutaramide
- le N-(2-mercapto-éthyl)-N'-(2-hydroxy-éthyl)-glutaramide
- le N-(2-mercapto-éthyl)-N',N'-(2,2-dihydroxy-éthyl)-glutaramide
- le N-(2-mercapto-éthyl)-N'-(3-diméthylamino-propyl)-glutaramide

6. Composition selon les revendications 1 ou 2, caractérisée par le fait que lorsque dans la formule générale (I) B représente le radical divalent $-(CH_2)_4-$, les composés sont choisis parmi :

- le N-(2-mercapto-éthyl)-adipamide
- le N-(2-mercapto-éthyl)-N'-méthyl-adipamide
- le N-(2-mercapto-éthyl)-N',N'-diméthyl-adipamide
- le N-(2-mercapto-éthyl)-N'-éthyl-adipamide
- le N-(2-mercapto-éthyl)-N'-(2-hydroxy-éthyl)-adipamide

- le N-(2-mercapto-éthyl)-N',N'-(2,2-dihydroxy-éthyl)-adipamide
- le N-(2-mercapto-éthyl)-N'-(3-diméthylamino-propyl)-adipamide

**7.** Composition selon les revendications 1 ou 2, caractérisée par le fait que lorsque dans la formule générale (I) B représente le radical divalent

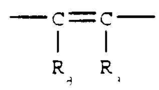

$R_8$ et $R_9$ ayant les mêmes significations que celles données à la revendication 1, les composés sont choisis parmi :

- le N-(2-mercapto-éthyl)-fumaramide
- le N-(3-mercapto-propyl)-fumaramide
- le N-(5-mercapto-pentyl)-fumaramide
- le N-[2-(2-mercapto-éthoxy)-éthyl]-fumaramide
- le N-(2-mercapto-éthyl)-N'-méthyl-fumaramide
- le N-(2-mercapto-éthyl)-N',N'-diméthyl-fumaramide
- le N-(2-mercapto-éthyl)-N'-éthyl-fumaramide
- le N-(2-mercapto-éthyl)-N',N'-diéthyl-fumaramide
- le N-(2-mercapto-éthyl)-N'-(2-hydroxy-éthyl)-fumaramide
- le N-(2-mercapto-éthyl)-N',N'-(2,2-dihydroxy-éthyl)-fumaramide
- le N-(2-mercapto-éthyl)-N'-(3-diméthylamino-propyl)-fumaramide
- le N-(2-mercapto-éthyl)-téréphtalamide
- le N-(2-mercapto-éthyl)-N'-méthyl-téréphtalamide
- le N-(2-mercapto-éthyl)-N'-éthyl-téréphtalamide
- le N-(2-mercapto-éthyl)-N'-(2-hydroxy-éthyl)-téréphtalamide
- le N-(2-mercapto-éthyl)-N'-(3-diméthylamino-propyl)-téréphtalamide

**8.** Composition selon les revendication 1 ou 2, caractérisée par le fait que lorsque dans la formule générale (I) B représente le radical divalent $-(CH_2)_m-$, m étant un nombre entier compris entre 6 et 7, les composés sont choisis parmi :

- l'acide octanedioïque 1-amide 5-[(2-mercapto-éthyl)-amide]
- l'acide nonanedioïque 1-amide 5-[(2-mercapto-éthyl)-amide]

**9.** Composition selon les revendications 1 ou 2, caractérisée par le fait que lorsque dans la formule générale (I) B représente le radical divalent

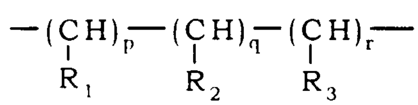

$R_1$, $R_2$, $R_3$, p, q, r ayant les mêmes significations que celles données à la revendication 1, les composés sont choisis parmi :

- l'acide 3-méthyl-pentanedioïque 1-amide 5-[(2-mercapto-éthyl)-amide]
- l'acide 2-éthyl-hexanedioïque 1-amide 6-[(2-mercapto-éthyl)-amide]

**10.** Composition selon les revendications 1 ou 2, caractérisée par le fait que lorsque dans la formule générale (I) B représente le radical divalent

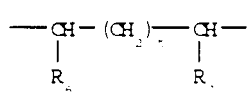

$R_6$, $R_7$, t ayant les mêmes significations que celles données à la revendication 1, les composés sont choisis parmi :

- l'acide 4-amino-pentanedioïque 1-amide 5-[(2-mercapto-éthyl)-amide]
- l'acide 3-amino-butanedioïque 1-amide 5-[(2-mercapto-éthyl)-amide]

**11.** Composition selon l'une quelconque des revendications 1 à 10, caractérisée par le fait que le composé de formule (I) est présent à une concentration comprise entre 0,5 et 30 % et de préférence entre 5 et 20 % en poids par rapport au poids total de la composition.

**12.** Composition selon l'une quelconque des revendications 1 à 11, caractérisée par le fait qu'elle présente un pH compris entre 4 et 11, et de préférence entre 6 et 10, obtenu soit à l'aide d'un agent alcalin choisi parmi l'ammoniaque, la monoéthanolamine, la diéthanolamine, la triéthanolamine, la propanediamine-1,3, le carbamate d'ammonium, un carbonate ou bicarbonate alcalin ou d'ammonium, un carbonate organique, un hydroxyde alcalin, soit à l'aide d'un agent acidifiant choisi parmi l'acide chlorhydrique, l'acide acétique, l'acide lactique, l'acide oxalique ou l'acide borique.

**13.** Composition selon l'une quelconque des revendications 1 à 12, caractérisée par le fait qu'elle contient en outre au moins un disulfure, la composition étant de type auto-neutralisante.

**14.** Composition selon la revendication 13, caractérisée par le fait que le disulfure correspond à la formule générale suivante :

$$\text{—(—S—A—NH—CO—B—CO—NRR')}_2 \qquad \text{(II)}$$

dans laquelle A, B, R et R' ont les mêmes significations que celles données pour la formule générale (I) de la revendication 1.

**15.** Composition selon les revendications 13 ou 14, caractérisée par le fait que le disulfure est présent en une proportion molaire par rapport au composé de formule (I) allant de 0,5 à 2,5, et de préférence de 1 à 2.

**16.** Procédé de déformation permanente des cheveux consistant, dans une première étape, à réduire les liaisons disulfures de la kératine par application d'une composition réductrice puis, dans une seconde étape, à reformer lesdites liaisons par application d'une composition oxydante, caractérisé par le fait que l'étape de réduction est réalisée à l'aide d'une composition cosmétique réductrice telle que définie dans l'une quelconque des revendications 1 à 15.

**17.** Procédé de préparation des composés de formule (I) tels que définis à la revendication 1, caractérisé par le fait qu'il consiste à faire réagir, dans une première étape et dans un solvant inerte, en présence d'un catalyseur, un aminothiol de formule $HS\text{-}A\text{-}NH_2$ ou son chlorhydrate sur un monoester de diacide de formule (2)

$$\text{HOOC—B—COOR}_{12}$$

A et B ayant les mêmes significations que celles données à la revendication 1 et $R_{12}$ est un radical alkyle ayant de 1 à 4 atomes de carbone, pour obtenir le N-mercaptoalkyl ester de formule (3) :

$$\text{HS—A—NH—CO—B—COOR}_{12} \qquad \text{(3)}$$

qui, dans une deuxième étape, est mis en réaction avec une amine de formule HNRR', R et R' ayant les mêmes significations que celles données à la revendication 1, éventuellement en présence d'un alcool inférieur.

**18.** Procédé selon la revendication 17 caractérisé par le fait que, dans la première étape, le solvant inerte est choisi parmi le dichlorométhane, le dichloro-1,2 éthane, le trichloro-1,1,1 éthane, le chloroforme, l'acétonitrile, le toluène, le dioxanne, le tétrahydrofuranne, le diméthoxy-1,2 éthane, le cyclohexane.

**19.** Procédé selon l'une quelconque des revendications 17 à 18, caractérisé par le fait que, dans la deuxième étape, le catalyseur est le dicyclohexyl carbodiimide.

**20.** Composés nouveaux, caractérisés par le fait qu'ils répondent à la formule générale (II) suivante :

$$-(-S-A-NH-CO-B-CO-NRR')_2 \qquad (II)$$

dans laquelle :

- A représente le radical divalent $-(CH_2)_n-$, n étant un nombre entier compris entre 2 et 5, ou le radical divalent $-(CH_2)_2-O-(CH_2)_2-$.
- B représente un radical choisi parmi le groupe comprenant :

  (a) le radical divalent $-(CH_2)_m-$, m étant un nombre entier compris entre 1 et 7,
  (b) le radical divalent

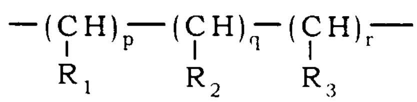

  dans lequel l'un des groupes $R_1$, $R_2$, $R_3$ représente un radical choisi parmi le groupe comprenant les radicaux alkyle, linéaires ou ramifiés, ayant de 1 à 4 atomes de carbone, méthoxyméthyle, phényle, benzyle, cyclohexyle, cyclopentyle et les deux autres groupes sont un atome d'hydrogène, p, q et r étant 0 ou un nombre entier compris entre 1 et 4 et $1 \leq p+q+r \leq 4$,
  (c) le radical divalent

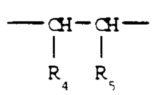

  dans lequel $R_4$ et $R_5$ soit, identiques ou différents, représentent un radical alkyle, linéaire ou ramifié, ayant de 1 à 4 atomes de carbone, soit forment ensemble, avec les atomes de carbone adjacents, un cycle cyclohexane ou cyclohexène,
  (d) le radical divalent

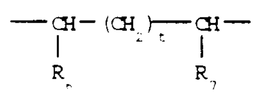

  dans lequel l'un des groupes $R_6$ ou $R_7$ représente un radical $-NH_2$, et l'autre groupe représentant un atome d'hydrogène t étant égal à 0 ou 1, et
  (e) le radical divalent

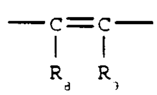

  dans lequel $R_8$ et $R_9$, soit, identiques ou différents, représentent un atome d'hydrogène, un radical alkyle, linéaire ou ramifié, ayant de 1 à 4 atomes de carbone, soit forment ensemble, avec les atomes de carbone adjacents, un cycle benzénique,

- R et R', identiques ou différents, représentent :

  . soit un atome d'hydrogène, ou un radical choisi parmi le groupe comprenant les radicaux alkyle, linéaires ou ramifiés, ayant de 1 à 4 atomes de carbone, les radicaux hydroxyalkyle, linéaires ou ramifiés, ayant de 1 à 5 atomes de carbone,
  . soit R désigne un atome d'hydrogène et R' un radical aminoalkyle $-(CH_2)_v-NR_{10}R_{11}$ dans lequel v est un nombre entier compris entre 1 et 3 et $R_{10}$ et $R_{11}$, identiques ou différents, représentent un atome d'hydrogène ou un radical alkyle, linéaire ou ramifié, ayant de 1 à 3 atomes de carbone, $R_{10}$ et $R_{11}$ ne pouvant simultanément représenter un atome d'hydrogène,

et les sels organiques et minéraux desdits composés de formule (II).

**21.** Composés nouveaux, caractérisés par le fait qu'ils répondent à la formule générale (III) suivante :

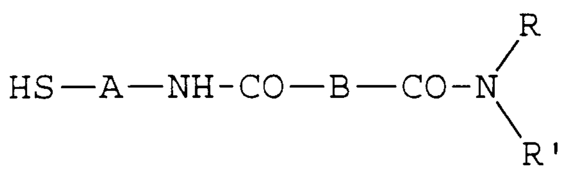

(III)

dans laquelle :

- A représente le radical divalent $-(CH_2)_n-$, n étant un nombre entier compris entre 2 et 5, ou le radical divalent $-(CH_2)_2-O-(CH_2)_2-$.
- B représente un radical choisi parmi le groupe comprenant :

(a) le radical divalent $-(CH_2)_m-$, m étant un nombre entier compris entre 1 et 7,
(b) le radical divalent

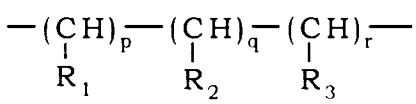

dans lequel l'un des groupes $R_1$, $R_2$, $R_3$ représente un radical choisi parmi le groupe comprenant les radicaux alkyle, linéaires ou ramifiés, ayant de 1 à 4 atomes de carbone, méthoxyméthyle, phényle, benzyle, cyclohexyle, cyclopentyle et les deux autres groupes sont un atome d'hydrogène, p, q et r étant 0 ou un nombre entier compris entre 1 et 4 et $1 \leq p + q + r \leq 4$,
(c) le radical divalent

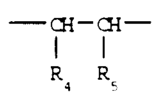

dans lequel $R_4$ et $R_5$ soit, identiques ou différents, représentent un radical alkyle, linéaire ou ramifié, ayant de 1 à 4 atomes de carbone, soit forment ensemble, avec les atomes de carbone adjacents, un cycle cyclohexane ou cyclohexène,
(d) le radical divalent

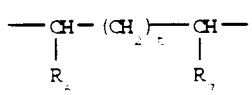

dans lequel l'un des groupes $R_6$ ou $R_7$ représente un radical $-NH_2$, et l'autre groupe représentant un atome d'hydrogène ; t étant égal à 0 ou 1, et
(e) le radical divalent

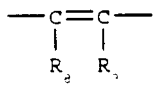

dans lequel $R_8$ et $R_9$, soit, identiques ou différents, représentent un atome d'hydrogène, un radical alkyle, linéaire ou ramifié, ayant de 1 à 4 atomes de carbone, soit forment ensemble, avec les atomes de carbone adjacents, un cycle benzénique.

- R et R', identiques ou différents, représentent :

  . soit un atome d'hydrogène, ou un radical choisi parmi le groupe comprenant les radicaux alkyle, linéaires ou ramifiés, ayant de 1 à 4 atomes de carbone, les radicaux hydroxyalkyle, linéaires ou ramifiés, ayant de 1 à 5 atomes de carbone,

  . soit R désigne un atome d'hydrogène et R' un radical aminoalkyle $-(CH_2)_v-NR_{10}R_{11}$ dans lequel v est un nombre entier compris entre 1 et 3 et $R_{10}$ et $R_{11}$, identiques ou différents, représentent un atome d'hydrogène ou un radical alkyle, linéaire ou ramifié, ayant de 1 à 3 atomes de carbone, $R_{10}$ et $R_{11}$ ne pouvant simultanément représenter un atome d'hydrogène,

et les sels organiques et minéraux desdits composés de formule (III)

à l'exclusion des composés dans lesquels :

soit

(i) A représente le radical divalent $-(CH_2)_2-$, et B représente le radical $-(CH_2)_m-$, m étant un nombre entier compris entre 2 et 7 ou le radical

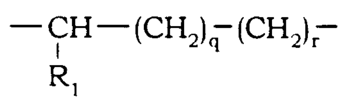

dans lequel $R_1$ représente un radical choisi parmi le groupe comprenant les radicaux alkyle, linéaires ou ramifiés, ayant de 1 à 4 atomes de carbone, phényle, benzyle, cyclohexyle, cyclopentyle, $1 < 1 + q + r \leq 4$,

et R et R', identiques ou différents, représentent :

  . soit un atome d'hydrogène, ou un radical choisi parmi le groupe comprenant les radicaux alkyle, linéaires ou ramifiés, ayant de 1 à 4 atomes de carbone,

  . soit R désigne un atome d'hydrogène et R' un radical aminoalkyle $-(CH_2)_v-NR_{10}R_{11}$ dans lequel v est un nombre entier compris entre 1 et 3 et $R_{10}$ et $R_{11}$, identiques ou différents, représentent un atome d'hydrogène ou un radical alkyle, linéaire ou ramifié, ayant de 1 à 3 atomes de carbone, $R_{10}$ et $R_{11}$ ne pouvant simultanément représenter un atome d'hydrogène,

soit

(ii) A représente le radical divalent $-(CH_2)_2-$ et B représente le radical $-CH_2-$, ou le radical

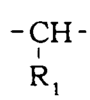

dans lequel $R_1$ représente un radical choisi parmi le groupe comprenant les radicaux alkyle, linéaires ou ramifiés, ayant de 1 à 4 atomes de carbone, méthoxyméthyle, phényle, benzyle,

et R et R', identiques ou différents, représentent :

  . soit un atome d'hydrogène, ou un radical choisi parmi le groupe comprenant les radicaux alkyle, linéaires ou ramifiés, ayant de 1 à 4 atomes de carbone,

  . soit R désigne un atome d'hydrogène et R' un radical aminoalkyle $-(CH_2)_v-NR_{10}R_{11}$ dans lequel v est un

nombre entier compris entre 1 et 3 et $R_{10}$ et $R_{11}$, identiques ou différents, représentent un radical alkyle, linéaire ou ramifié, ayant de 1 à 3 atomes de carbone,

**22.** Composés selon la revendication 21, caractérisés par le fait que les sels des composés de formule (III) sont choisis parmi les chlorhydrates, bromhydrates, citrates, oxalates, et acétates.

**23.** Composés selon la revendication 21, caractérisés par le fait que lorsque dans la formule générale (III), B représente le radical divalent $-CH_2-$, les composés sont choisis parmi :

- le N-(3-mercapto-propyl)-malonamide
- le N-(5-mercapto-pentyl)-malonamide
- le N-[2-(2-mercapto-éthoxy)-éthyl]-malonamide
- le N-(2-mercapto-éthyl)-N'-(2-hydroxy-éthyl)-malonamide
- le N-(2-mercapto-éthyl)-N',N'-(2,2-dihydroxy-éthyl)-malonamide

**24.** Composés selon la revendication 21, caractérisés par le fait que lorsque dans la formule générale (III) B représente le radical divalent $-(CH_2)_2-$, les composés sont choisis parmi :

- le N-(3-mercapto-propyl)-succinamide
- le N-(5-mercapto-pentyl)-succinamide
- le N-[2-(2-mercapto-éthoxy)-éthyl]-succinamide

**25.** Composés selon la revendication 21, caractérisés par le fait que lorsque dans la formule générale (III) B représente le radical divalent $-(CH_2)_3-$, les composés sont choisis parmi :

- le N-(3-mercapto-propyl)-glutaramide
- le N-(5-mercapto-pentyl)-glutaramide
- le N-[2-(2-mercapto-éthoxy)-éthyl]-glutaramide

**26.** Composés selon la revendication 21, caractérisés par le fait que lorsque dans la formule générale (III) B représente le radical divalent

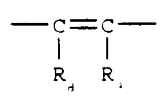

$R_8$ et $R_9$ ayant les mêmes significations que celles données à la revendication 21, les composés sont choisis parmi :

- le N-(2-mercapto-éthyl)-fumaramide
- le N-(3-mercapto-propyl)-fumaramide
- le N-(5-mercapto-pentyl)-fumaramide
- le N-[2-(2-mercapto-éthoxy)-éthyl]-fumaramide
- le N-(2-mercapto-éthyl)-N'-méthyl-fumaramide
- le N-(2-mercapto-éthyl)-N',N'-diméthyl-fumaramide
- le N-(2-mercapto-éthyl)-N'-éthyl-fumaramide
- le N-(2-mercapto-éthyl)-N',N'-diéthyl-fumaramide
- le N-(2-mercapto-éthyl)-N'-(2-hydroxy-éthyl)-fumaramide
- le N-(2-mercapto-éthyl)-N',N'-(2,2-dihydroxy-éthyl)-fumaramide
- le N-(2-mercapto-éthyl)-N'-(3-diméthylamino-propyl)-fumaramide
- le N-(2-mercapto-éthyl)-téréphtalamide
- le N-(2-mercapto-éthyl)-N'-méthyl-téréphtalamide
- le N-(2-mercapto-éthyl)-N'-éthyl-téréphtalamide
- le N-(2-mercapto-éthyl)-N'-(2-hydroxy-éthyl)-téréphtalamide
- le N-(2-mercapto-éthyl)-N'-(3-diméthylamino-propyl)-téréphtalamide

**27.** Composés selon la revendication 21, caractérisés par le fait que lorsque dans la formule générale (III) B représente

le radical

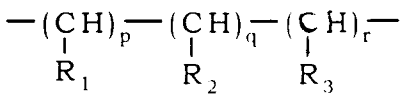

divalent $R_1$, $R_2$, $R_3$, p, q, r ayant les mêmes significations que celles données à la revendication 21, les composés sont choisis parmi :

- l'acide 3-méthyl-pentanedioïque 1-amide 5-[(2-mercapto-éthyl)-amide]
- l'acide 2-éthyl-hexanedioïque 1-amide 6-[(2-mercapto-éthyl)-amide]

**28.** Composés selon la revendication 21, caractérisés par le fait que lorsque dans la formule générale (III) B représente le radical divalent

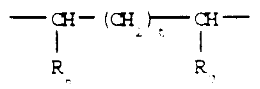

$R_6$, $R_7$, t ayant les mêmes significations que celles données à la revendication 21, les composés sont choisis parmi: :

- l'acide 4-amino-pentanedioïque 1-amide 5-[(2-mercapto-éthyl)-amide]
- l'acide 3-amino-butanedioïque 1-amide 5-[(2-mercapto-éthyl)-amide]

**Claims**

**1.** Cosmetic composition for the first step of an operation for permanent deformation of hair, consisting in reducing the disulphide bonds of keratin, characterized in that it contains at least one compound which has the following general formula:

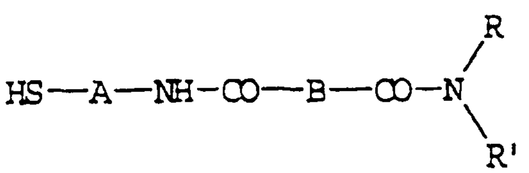

(I)

in which

- A denotes the divalent radical $-(CH_2)_n-$, n being an integer between 2 and 5, or the divalent radical $-(CH_2)_2-O-(CH_2)_2-$,
- B denotes a radical chosen from the group including:

  (a) the divalent radical $-(CH_2)_m-$, m being an integer between 1 and 7,
  (b) the divalent radical

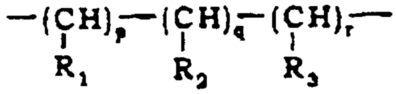

in which one of the groups $R_1$, $R_2$ and $R_3$ denotes a radical chosen from the group including linear or branched alkyl radicals containing from 1 to 4 carbon atoms, methoxymethyl, phenyl, benzyl, cyclohexyl and cyclopentyl radicals, and the other two groups are a hydrogen atom, p, q and r being 0 or an integer

between 1 and 4 and $1 \le p + q + r \le 4$,
(c) the divalent radical

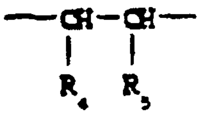

in which $R_4$ and $R_5$ either, being identical or different, denote a linear or branched alkyl radical containing from 1 to 4 carbon atoms, or, together with the adjacent carbon atoms, form a cyclohexane or cyclohexene ring,
(d) the divalent radical

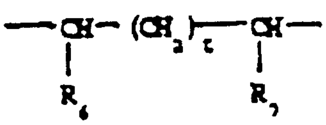

in which one of the groups $R_6$ and $R_7$ denotes an $-NH_2$ radical and the other group denoting a hydrogen atom; t being equal to 0 or 1, and
(e) the divalent radical

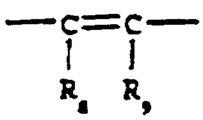

in which $R_8$ and $R_9$ either, being identical or different, denote a hydrogen atom or a linear or branched alkyl radical containing from 1 to 4 carbon atoms or, together with the adjacent carbon atoms, form a benzene ring.

- R and R', which are identical or different, denote:
  - either a hydrogen atom or a radical chosen from the group including linear or branched alkyl radicals containing from 1 to 4 carbon atoms, and linear or branched hydroxyalkyl radicals containing from 1 to 5 carbon atoms,
  - or R denotes a hydrogen atom and R' an aminoalkyl radical $-(CH_2)_v-NR_{10}R_{11}$ in which v is an integer between 1 and 3 and $R_{10}$ and $R_{11}$, which are identical or different, denote a hydrogen atom or a linear or branched alkyl radical containing from 1 to 3 carbon atoms, $R_{10}$ and $R_{11}$ being incapable of simultaneously denoting a hydrogen atom,

and the organic and inorganic salts of the said compounds of formula (I).

**2.** Composition according to Claim 1, characterized in that the salts of the compounds of formula (I) are chosen from hydrochlorides, hydrobromides, citrates, oxalates and acetates.

**3.** Composition according to Claim 1 or 2, characterized in that when, in the general formula (I), B denotes the $-CH_2-$ divalent radical, the compounds are chosen from:

- N-(2-mercaptoethyl)malonamide
- N-(3-mercaptopropyl)malonamide
- N-(5-mercaptopentyl)malonamide
- N-[2-(2-mercaptoethoxy)ethyl]malonamide
- N-(2-mercaptoethyl)-N'-methylmalonamide
- N-(2-mercaptoethyl)-N',N'-dimethylmalonamide

- N-(2-mercaptoethyl)-N'-ethylmalonamide
- N-(2-mercaptoethyl)-N',N'-diethylmalonamide
- N-(2-mercaptoethyl)-N'-(2-hydroxyethyl)malonamide
- N-(2-mercaptoethyl)-N',N'-(2,2-dihydroxyethyl)malonamide
- N-(2-mercaptoethyl)-N'-(3-dimethylaminopropyl)malonamide
- N-(2-mercaptoethyl)-N'-(3-diethylaminopropyl)malonamide.

**4.** Composition according to Claim 1 or 2, characterized in that when, in the general formula (I), B denotes the $-(CH_2)_2-$ divalent radical, the compounds are chosen from:

- N-(2-mercaptoethyl)succinamide
- N-(3-mercaptopropyl)succinamide
- N-(5-mercaptopentyl)succinamide
- N-[2-(2-mercaptoethoxy)ethyl]succinamide
- N-(2-mercaptoethyl)-N'-methylsuccinamide
- N-(2-mercaptoethyl)-N',N'-dimethylsuccinamide
- N-(2-mercaptoethyl)-N'-ethylsuccinamide
- N-(2-mercaptoethyl)-N',N'-diethylsuccinamide
- N-(2-mercaptoethyl)-N'-(2-hydroxyethyl)succinamide
- N-(2-mercaptoethyl)-N',N'-(2,2-dihydroxyethyl)succinamide
- N-(2-mercaptoethyl)-N'-(3-dimethylaminopropyl)succinamide
- N-(2-mercaptoethyl)-N'-(3-diethylaminopropyl)succinamide.

**5.** Composition according to Claim 1 or 2, characterized in that when, in the general formula (I), B denotes the $-(CH_2)_3-$ divalent radical, the compounds are chosen from:

- N-(2-mercaptoethyl)glutaramide
- N-(3-mercaptopropyl)glutaramide
- N-(5-mercaptopentyl)glutaramide
- N-[2-(2-mercaptoethoxy)ethyl]glutaramide
- N-(2-mercaptoethyl)-N'-methylglutaramide
- N-(2-mercaptoethyl)-N',N'-dimethylglutaramide
- N-(2-mercaptoethyl)-N'-ethylglutaramide
- N-(2-mercaptoethyl)-N',N'-diethylglutaramide
- N-(2-mercaptoethyl)-N'-(2-hydroxyethyl)glutaramide
- N-(2-mercaptoethyl)-N',N'-(2,2-dihydroxyethyl)glutaramide
- N-(2-mercaptoethyl)-N'-(3-dimethylaminopropyl)glutaramide.

**6.** Composition according to Claim 1 or 2, characterized in that when, in the general formula (I), B denotes the $-(CH_2)_4-$ divalent radical, the compounds are chosen from:

- N-(2-mercaptoethyl)adipamide
- N-(2-mercaptoethyl)-N'-methyladipamide
- N-(2-mercaptoethyl)-N',N'-dimethyladipamide
- N-(2-mercaptoethyl)-N'-ethyladipamide
- N-(2-mercaptoethy)-N'-(2-hydroxyethyl)adipamide
- N-(2-mercaptoethyl)-N',N'-(2,2-dihydroxyethyl)adipamide
- N-(2-mercaptoethyl)-N'-(3-dimethylaminopropyl)adipamide.

**7.** Composition according to Claim 1 or 2, characterized in that when, in the general formula (I), B denotes the divalent radical

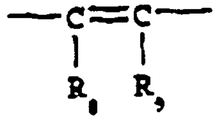

$R_8$ and $R_9$ having the same meanings as those given in Claim 1, the compounds are chosen from:

- N-(2-mercaptoethyl)fumaramide
- N-(3-mercaptopropyl)fumaramide
- N-(5-mercaptopentyl)fumaramide
- N-[2-(2-mercaptoethoxy)ethyl]fumaramide
- N-(2-mercaptoethyl)-N'-methylfumaramide
- N-(2-mercaptoethyl)-N',N'-dimethylfumaramide
- N-(2-mercaptoethyl)-N'-ethylfumaramide
- N-(2-mercaptoethyl)-N',N'-diethylfumaramide
- N-(2-mercaptoethyl)-N'-(2-hydroxyethyl)fumaramide
- N-(2-mercaptoethyl)-N',N'-(2,2-dihydroxyethyl)fumaramide
- N-(2-mercaptoethyl)-N'-(3-dimethylaminopropyl)fumaramide;
- N-(2-mercaptoethyl)terephthalamide
- N-(2-mercaptoethyl)-N'-methylterephthalamide
- N-(2-mercaptoethyl)-N'-ethylterephthalamide
- N-(2-mercaptoethyl)-N'-(2-hydroxyethyl)terephthalamide
- N-(2-mercaptoethyl)-N'-(3-dimethylaminopropyl)terephthalamide.

**8.** Composition according to Claim 1 or 2, characterized in that when, in the general formula (I), B denotes the divalent radical $-(CH_2)_m-$, m being an integer between 6 and 7, the compounds are chosen from:

- octanedioic acid 1-amide 5-[(2-mercaptoethyl)amide]
- nonanedioic acid 1-amide 5-[(2-mercaptoethyl)amide].

**9.** Composition according to Claim 1 or 2, characterized in that when, in the general formula (I), B denotes the divalent radical

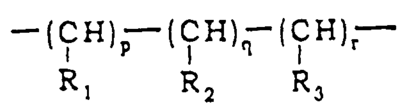

$R_1$, $R_2$, $R_3$, p, q and r having the same meanings as those given in Claim 1, the compounds are chosen from:

- 3-methylpentanedioic acid 1-amide 5-[(2-mercaptoethyl)amide]
- 2-ethylhexanedioic acid 1-amide 6-[(2-mercaptoethyl)amide].

**10.** Composition according to Claim 1 or 2, characterized in that when, in the general formula (I), B denotes the divalent radical

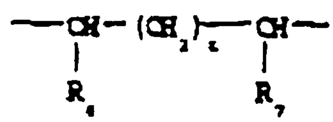

$R_6$, $R_7$ and t having the same meanings as those given in Claim 1, the compounds are chosen from:

- 4-aminopentanedioic acid 1-amide 5-[(2-mercaptoethyl)amide]
- 3-aminobutanedioic acid 1-amide 5-[(2-mercaptoethyl)amide].

**11.** Composition according to any one of Claims 1 to 10, characterized in that the compound of formula (I) is present in a concentration of between 0.5 and 30 % and preferably between 5 and 20 % by weight relative to the total weight of the composition.

**12.** Composition according to any one of Claims 1 to 11, characterized in that it has a pH of between 4 and 11 and preferably between 6 and 10 obtained either with the aid of an alkaline agent chosen from aqueous ammonia, monoethanolamine, diethanolamine, triethanolomine, 1,3-propanediamine, ammonium carbamate, an alkali metal or ammonium carbonate or bicarbonate, an organic carbonate, an alkali metal hydroxide, or with the aid of an acidifying agent chosen from hydrochloric acid, acetic acid, lactic acid, oxalic acid and boric acid.

**13.** Composition according to any one of Claims 1 to 12, characterized in that it additionally contains at least one disulphide, the composition being of the self-neutralizing type.

**14.** Composition according to Claim 13, characterized in that the disulphide corresponds to the following general formula:

$$—(—S—A—NH—CO—B—CO—NRR')_2 \qquad (II)$$

in which A, B, R and R' have the same meanings as those given for the general formula (I) of Claim 1.

**15.** Composition according to Claim 13 or 14, characterized in that the disulphide is present in a molar proportion, relative to the compound of formula (I), ranging from 0.5 to 2.5 and preferably from 1 to 2.

**16.** Process for permanent deformation of hair consisting, in a first stage, in reducing the disulphide bonds of keratin by application of a reducing composition and then, in a second step, in reforming the said bonds by application of an oxidizing composition, characterized in that the reduction step is carried out with the aid of a reducing cosmetic composition as defined in any one of Claims 1 to 15.

**17.** Process for the preparation of the compounds of formula (I) as defined in Claim 1, characterized in that it consists in reacting, in a first stage and in an inert solvent, in the presence of a catalyst, an aminothiol of formula $HS\text{-}A\text{-}NH_2$ or its hydrochloride with a monoester of a diacid of formula (2)

$$HOOC—B—COOR_{12}$$

A and B having the same meanings as those given in Claim 1, and $R_{12}$ is an alkyl radical containing from 1 to 4 carbon atoms, to obtain the N-mercaptoalkyl ester of formula (3):

$$HS—A—NH—CO—B—COOR_{12} \qquad (3)$$

which, in a second stage, is reacted with an amine of formula HNRR', R and R' having the same meanings as those given in Claim 1, optionally in the presence of a lower alcohol.

**18.** Process according to Claim 17, characterized in that, in the first stage, the inert solvent is chosen from dichloromethane, 1,2-dichloro-ethane, 1,1,1-trichloroethane, chloroform, acetonitrile, toluene, dioxane, tetrahydrofuran, 1,2-dimethoxyethane and cyclohexane.

**19.** Process according to any one of Claims 17 to 18, characterized in that, in the second stage, the catalyst is dicyclohexylcarbodiimide.

**20.** New compounds characterized in that they correspond to the following general formula (II):

$$—(—S—A—NH—CO—B—CO—NRR')_2 \qquad (II)$$

in which

- A denotes the divalent radical $-(CH_2)_n-$, n being an integer between 2 and 5, or the divalent radical $-(CH_2)_2-O-(CH_2)_2-$,
- B denotes a radical chosen from the group including:

  (a) the divalent radical $-(CH_2)_m-$, m being an integer between 1 and 7,
  (b) the divalent radical

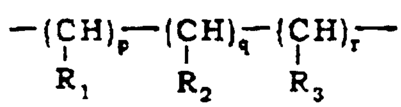

in which one of the groups $R_1$, $R_2$ and $R_3$ denotes a radical chosen from the group including linear or branched alkyl radicals containing from 1 to 4 carbon atoms, methoxymethyl, phenyl, benzyl, cyclohexyl and cyclopentyl radicals, and the other two groups are a hydrogen atom, p, q and r being 0 or an integer between 1 and 4 and $1 \leq p + q + r \leq 4$,

(c) the divalent radical

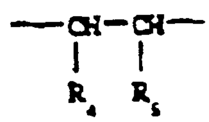

in which $R_4$ and $R_5$ either, being identical or different, denote a linear or branched alkyl radical containing from 1 to 4 carbon atoms, or, together with the adjacent carbon atoms, form a cyclohexane or cyclohexene ring,

(d) the divalent radical

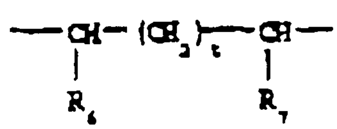

in which one of the groups $R_6$ and $R_7$ denotes an $-NH_2$ radical and the other group denoting a hydrogen atom; t being equal to 0 or 1, and

(e) the divalent radical

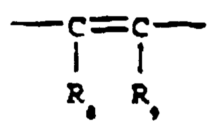

in which $R_8$ and $R_9$ either, being identical or different, denote a hydrogen atom or a linear or branched alkyl radical containing from 1 to 4 carbon atoms or, together with the adjacent carbon atoms, form a benzene ring,

- R and R', which are identical or different, denote:
  - either a hydrogen atom or a radical chosen from the group including linear or branched alkyl radicals containing from 1 to 4 carbon atoms, and linear or branched hydroxyalkyl radicals containing from 1 to 5 carbon atoms,
  - or R denotes a hydrogen atom and R' an aminoalkyl radical $-(CH_2)_v-NR_{10}R_{11}$ in which v is an integer between 1 and 3 and $R_{10}$ and $R_{11}$, which are identical or different, denote a hydrogen atom or a linear or branched alkyl radical containing from 1 to 3 carbon atoms, $R_{10}$ and $R_{11}$ being incapable of simultaneously denoting a hydrogen atom,

and the organic and inorganic salts of the said compounds of formula (II).

**21.** New compounds characterized in that they correspond to the following general formula (III):

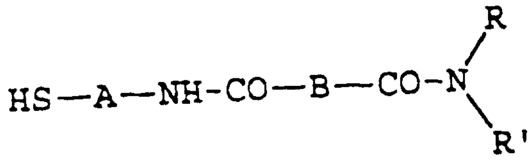

(III)

in which

- A denotes the divalent radical $-(CH_2)_n-$, n being an integer between 2 and 5, or the divalent radical $-(CH_2)_2-O-(CH_2)_2-$,
- B denotes a radical chosen from the group including:

  (a) the divalent radical $-(CH_2)_m-$, m being an integer between 1 and 7,
  (b) the divalent radical

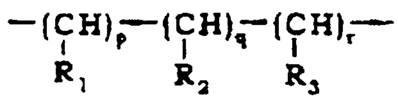

  in which one of the groups $R_1$, $R_2$ and $R_3$ denotes a radical chosen from the group including linear or branched alkyl radicals containing from 1 to 4 carbon atoms, methoxymethyl, phenyl, benzyl, cyclohexyl and cyclopentyl radicals, and the other two groups are a hydrogen atom, p, q and r being 0 or an integer between 1 and 4 and $1 \leq p + q + r \leq 4$,
  (c) the divalent radical

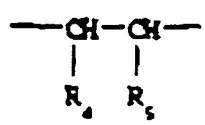

  in which $R_4$ and $R_5$ either, being identical or different, denote a linear or branched alkyl radical containing from 1 to 4 carbon atoms, or, together with the adjacent carbon atoms, form a cyclohexane or cyclohexene ring,
  (d) the divalent radical

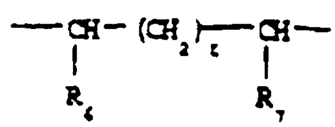

  in which one of the groups $R_6$ and $R_7$ denotes an $-NH_2$ radical and the other group denoting a hydrogen atom; t being equal to 0 or 1, and
  (e) the divalent radical

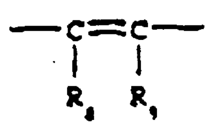

in which $R_8$ and $R_9$ either, being identical or different, denote a hydrogen atom or a linear or branched alkyl radical containing from 1 to 4 carbon atoms or, together with the adjacent carbon atoms, form a benzene ring,

- R and R', which are identical or different, denote:
  - either a hydrogen atom or a radical chosen from the group including linear or branched alkyl radicals containing from 1 to 4 carbon atoms, and linear or branched hydroxyalkyl radicals containing from 1 to 5 carbon atoms,
  - or R denotes a hydrogen atom and R' an aminoalkyl radical $-(CH_2)_v-NR_{10}R_{11}$ in which v is an integer between 1 and 3 and $R_{10}$ and $R_{11}$, which are identical or different, denote a hydrogen atom or a linear or branched alkyl radical containing from 1 to 3 carbon atoms, $R_{10}$ and $R_{11}$ being incapable of simultaneously denoting a hydrogen atom,

and the organic and inorganic salts of the said compounds of formula (III)
excluding the compounds in which:
either

(i) A denotes the divalent radical $-(CH_2)_2-$ and B denotes the radical $-(CH_2)_m-$, m being an integer between 2 and 7
or the radical

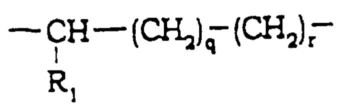

in which $R_1$ denotes a radical chosen from the group including linear or branched alkyl radicals containing from 1 to 4 carbon atoms, phenyl, benzyl, cyclohexyl and cyclopentyl radicals, $1 < 1 + q + r \leq 4$, and R and R', which are identical or different, denote:

- either a hydrogen atom or a radical chosen from the group including linear or branched alkyl radicals containing from 1 to 4 carbon atoms,
- or R denotes a hydrogen atom and R' an aminoalkyl radical $-(CH_2)_v-NR_{10}R_{11}$ in which v is an integer between 1 and 3 and $R_{10}$ and $R_{11}$, which are identical or different, denote a hydrogen atom or a linear or branched alkyl radical containing from 1 to 3 carbon atoms, $R_{10}$ and $R_{11}$ not being able simultaneously to denote a hydrogen atom,

or
(ii) A denotes the divalent radical $-(CH_2)_2-$ and B denotes the radical $-CH_2-$,
or the radical

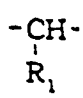

in which $R_1$ denotes a radical chosen from the group including linear or branched alkyl radicals containing from 1 to 4 carbon atoms and methoxymethyl, phenyl and benzyl radicals,
and R and R', which are identical or different, denote:

- either a hydrogen atom or a radical chosen from the group including linear or branched alkyl radicals containing from 1 to 4 carbon atoms,
- or R denotes a hydrogen atom and R' an aminoalkyl radical $-(CH_2)_v-NR_{10}R_{11}$ in which v is an integer between 1 and 3 and $R_{10}$ and $R_{11}$, which are identical or different, denote a linear or branched alkyl radical containing from 1 to 3 carbon atoms.

22. Compounds according to Claim 21, characterized in that the salts of the compounds of formula (III) are chosen from hydrochlorides, hydrobromides, citrates, oxalates and acetates.

23. Compounds according to Claim 21, characterized in that when, in the general formula (III), B denotes the $-CH_2-$ divalent radical the compounds are chosen from:

- N-(3-mercaptopropyl)malonamide
- N-(5-mercaptopentyl)malonamide
- N-[2-(2-mercaptoethoxy)ethyl]malonamide
- N-(2-mercaptoethyl)-N'-(2-hydroxyethyl)malonamide
- N-(2-mercaptoethyl)-N',N'-(2,2-dihydroxyethyl)malonamide.

24. Compounds according to Claim 21, characterized in that when, in the general formula (III), B denotes the $-(CH_2)_2-$ divalent radical the compounds are chosen from:

- N-(3-mercaptopropyl)succinamide
- N-(5-mercaptopentyl)succinamide
- N-[2-(2-mercaptoethoxy)ethyl]succinamide.

25. Compounds according to Claim 21, characterized in that when, in the general formula (III), B denotes the $-(CH_2)_3-$ divalent radical the compounds are chosen from:

- N-(3-mercaptopropyl)glutaramide
- N-(5-mercaptopentyl)glutaramide
- N-[2-(2-mercaptoethoxy)ethyl]glutaramide.

26. Compounds according to Claim 21, characterized in that when, in the general formula (III), B denotes the divalent radical

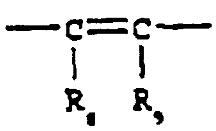

$R_8$ and $R_9$ having the same meanings as those given in Claim 21, the compounds are chosen from:

- N-(2-mercaptoethyl)fumaramide
- N-(3-mercaptopropyl)fumaramide
- N-(5-mercaptopentyl)fumaramide
- N-[2-(2-mercaptoethoxy)ethyl]fumaramide
- N-(2-mercaptoethyl)-N'-methylfumaramide
- N-(2-mercaptoethyl)-N',N'-dimethylfumaramide
- N-(2-mercaptoethyl)-N'-ethylfumaramide
- N-(2-mercaptoethyl)-N',N'-diethylfumaramide
- N-(2-mercaptoethyl)-N'-(2-hydroxyethyl)fumaramide
- N-(2-mercaptoethyl)-N',N'-(2,2-dihydroxyethyl)fumaramide
- N-(2-mercaptoethyl)-N'-(3-dimethylaminopropyl)fumaramide;
- N-(2-mercaptoethyl)terephthalamide
- N-(2-mercaptoethyl)-N'-methylterephthalamide
- N-(2-mercaptoethyl)-N'-ethylterephthalamide
- N-(2-mercaptoethyl)-N'-(2-hydroxyethyl)terephthalamide
- N-(2-mercaptoethyl)-N'-(3-dimethylaminopropyl)terephthalamide.

27. Compounds according to Claim 21, characterized in that when, in the general formula (III), B denotes the divalent radical

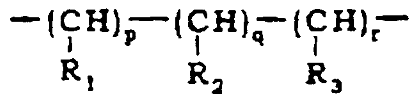

$R_1$, $R_2$, $R_3$, p, q and r having the same meanings as those given in Claim 21, the compounds are chosen from:

- 3-methylpentanedioic acid 1-amide 5-[(2-mercaptoethyl)amide]
- 2-ethylhexanedioic acid 1-amide 6-[(2-mercaptoethyl)amide].

**28.** Compounds according to Claim 21, characterized in that when, in the general formula (III), B denotes the divalent radical

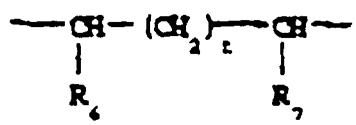

$R_6$, $R_7$ and t having the same meanings as those given in Claim 21, the compounds are chosen from:

- 4-aminopentanedioic acid 1-amide 5-[(2-mercaptoethyl)amide]
- 3-aminobutanedioic acid 1-amide 5-[(2-mercaptoethyl)amide].

**Patentansprüche**

**1.** Kosmetische Zusammensetzung für den ersten. Schritt einer permanenten Verformung des Haares, der darin besteht, die Disulfidbindungen des Keratins zu reduzieren,
**dadurch gekennzeichnet, daß**
sie mindestens eine Verbindung der folgenden allgemeinen Formel enthält:

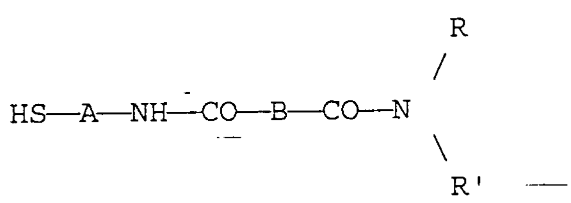

(I),

worin bedeuten:

- A eine zweiwertige Gruppe $-(CH_2)_n-$, wobei n eine ganze Zahl im Bereich von 2 bis 5 ist, oder die zweiwertige Gruppe $-(CH_2)_2-O-(CH_2)_2-$,
- B eine Gruppe, die ausgewählt ist unter:

  (a) einer zweiwertigen Gruppe $-(CH_2)_m-$, wobei m eine ganze Zahl im Bereich von 1 bis 7 ist,

  (b) einer zweiwertigen Gruppe

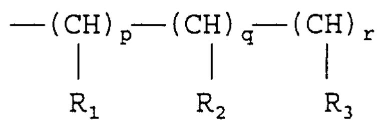

worin eine der Gruppen $R_1$, $R_2$, $R_3$ unter den geradkettigen oder verzweigten Alkylgruppen mit 1 bis 4 Kohlenstoffatomen, Methoxymethyl, Phenyl, Benzyl, Cyclohexyl oder Cyclopentyl ausgewählt ist und die beiden anderen Gruppen Wasserstoff bedeuten, wobei p, q und r Null oder eine ganze Zahl im Bereich von 1 bis 4 bedeuten und ferner gilt:

$$1 \leq p + q + r \leq 4,$$

(c) einer zweiwertigen Gruppe

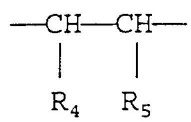

worin $R_4$ oder $R_5$, die identisch oder voneinander verschieden sind, eine geradkettige oder verzweigte Alkylgruppe mit 1 bis 4 Kohlenstoffatomen bedeuten oder gemeinsam mit den angrenzenden Kohlenstoffatomen einen Cyclohexan- oder Cyclohexenring bilden,

(d) einer zweiwertigen Grupp

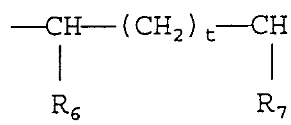

worin eine der Gruppen $R_6$ oder $R_7$ die Gruppe $-NH_2$ und die andere Gruppe ein Wasserstoffatom bedeutet, wobei t Null oder 1 ist, und

(e) einer zweiwertigen Gruppe

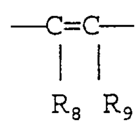

worin $R_8$ und $R_9$, die identisch oder voneinander verschieden sind, Wasserstoff oder eine geradkettige oder verzweigte Alkylgruppe mit 1 bis 4 Kohlenstoffatomen bedeuten oder gemeinsam mit den angrenzenden Kohlenstoffatomen einen Benzolring bilden.

- R und R', die identisch oder voneinander verschieden sind:

  . Wasserstoff oder eine Gruppe, die ausgewählt ist unter den geradkettigen oder verzweigten Alkylgruppen mit 1 bis 4 Kohlenstoffatomen und den geradkettigen oder verzweigten Hydroxyalkylgruppen mit 1 bis 5 Kohlenstoffatomen, oder

. R bedeutet Wasserstoff und R' eine Aminoalkylgruppe $-(CH_2)_v-NR_{10}R_{11}$, worin v eine ganze Zahl von 1 bis 3 bedeutet und $R_{10}$ und $R_{11}$, die identisch oder voneinander verschieden sind, Wasserstoff oder eine geradkettige oder verzweigte Alkylgruppe mit 1 bis 3 Kohlenstoffatomen bedeuten, wobei $R_{10}$ und $R_{11}$ nicht gleichzeitig Wasserstoff bedeuten können,

und die organischen und anorganischen Salze der Verbindungen der Formel (I).

**2.** Zusammensetzung nach Anspruch 1, dadurch gekennzeichnet, daß die Salze der Verbindungen der Formel (I) unter den Hydrochloriden, Hydrobromiden, Citraten, Oxalaten und Acetaten ausgewählt sind.

**3.** Zusammensetzung nach den Ansprüchen 1 oder 2, dadurch gekennzeichnet, daß, wenn in der allgemeinen Formel (I) B die zweiwertige Gruppe $-CH_2-$ bedeutet, die Verbindungen ausgewählt sind unter:

- N-(2-Mercaptoethyl)-malonamid
- N-(3-Mercaptopropyl)-malonamid
- N-(5-Mercaptopentyl)-malonamid
- N-[2-(2-Mercaptoethoxy)-ethyl]-malonamid
- N-(2-Mercaptoethyl)-N'-methyl-malonamid
- N-(2-Mercaptoethyl)-N',N'-dimethyl-malonamid
- N-(2-Mercaptoethyl)-N'-ethyl-malonamid
- N-(2-Mercaptoethyl)-N',N'-diethyl-malonamid
- N-(2-Mercaptoethyl)-N'-(2-hydroxyethyl)-malonamid
- N-(2-Mercaptoethyl)-N',N'-(2,2-dihydroxyethyl)-malonamid
- N-(2-Mercaptoethyl)-N'-(3-dimethylaminopropyl)-malonamid
- N-(2-Mercaptoethyl)-N'-(3-diethylaminopropyl)-malonamid

**4.** Zusammensetzung nach den Ansprüchen 1 oder 2, dadurch gekennzeichnet, daß, wenn in der allgemeinen Formel (I) B die zweiwertige Gruppe $-(CH_2)_2-$ bedeutet, die Verbindungen ausgewählt sind unter:

- N-(2-Mercaptoethyl)-succinamid
- N-(3-Mercaptopropyl)-succinamid
- N-(5-Mercaptopentyl)-succinamid
- N-[2-(2-Mercaptoethoxy)-ethyl]-succinamid
- N-(2-Mercaptoethyl)-N'-methyl-succinamid
- N-(2-Mercaptoethyl)-N,N'-dimethyl-succinamid
- N-(2-Mercaptoethyl)-N'-ethyl-succinamid
- N-(2-Mercaptoethyl)-N',N'-diethyl-succinamid
- N-(2-Mercaptoethyl)-N'-(2-hydroxyethyl)-succinamid
- N-(2-Mercaptoethyl)-N',N'-(2,2-dihydroxyethyl)-succinamid
- N-(2-Mercaptoethyl)-N'-(3-dimethylaminopropyl)-succinamid
- N-(2-Mercaptoethyl)-N'-(3-diethylaminopropyl)-succinamid

**5.** Zusammensetzung nach den Ansprüchen 1 oder 2, dadurch gekennzeichnet, daß, wenn in der allgemeinen Formel (I) B die zweiwertige Gruppe $-(CH_2)_3-$ bedeutet, die Verbindungen ausgewählt sind unter:

- N-(2-Mercaptoethyl)-glutaramid
- N-(3-Mercaptopropyl)-glutaramid
- N-(5-Mercaptopentyl)-glutaramid
- N-[2-(2-Mercaptoethoxy)-ethyl]-glutaramid
- N-(2-Mercaptoethyl)-N'-methyl-glutaramid
- N-(2-Mercaptoethyl)-N',N'-dimethyl-glutaramid
- N-(2-Mercaptoethyl)-N'-ethyl-glutaramid
- N-(2-Mercaptoethyl)-N',N'-diethyl-glutaramid
- N-(2-Mercaptoethyl)-N'-(2-hydroxyethyl)-glutaramid
- N-(2-Mercaptoethyl)-N',N'-(2,2-dihydroxyethyl)-glutaramid
- N-(2-Mercaptoethyl)-N'-(3-dimethylaminopropyl)-glutaramid

**6.** Zusammensetzung nach den Ansprüchen 1 oder 2, dadurch gekennzeichnet, daß, wenn in der allgemeinen Formel (I) B die zweiwertige Gruppe $-(CH_2)_4-$ bedeutet, die Verbindungen ausgewählt sind unter:

- N-(2-Mercaptoethyl)-adipamid
- N-(2-Mercaptoethyl)-N'-methyl-adipamid
- N-(2-Mercaptoethyl)-N',N'-dimethyl-adipamid
- N-(2-Mercaptoethyl)-N'-ethyl-adipamid
- N-(2-Mercaptoethyl)-N'-(2-hydroxyethyl)-adipamid
- N-(2-Mercaptoethyl)-N',N'-(2,2-dihydroxyethyl)-adipamid
- N-(2-Mercaptoethyl)-N'-(3-dimethylaminopropyl)-adipamid

**7.** Zusammensetzung nach den Ansprüchen 1 oder 2, dadurch gekennzeichnet, daß, wenn in der allgemeinen Formel (I) B die zweiwertige Gruppe

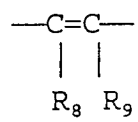

bedeutet, wobei $R_8$ und $R_9$ die für Anspruch 1 angegebenen Bedeutungen aufweisen, die Verbindungen ausgewählt sind unter:

- N-(2-Mercaptoethyl)-fumaramid
- N-(3-Mercaptopropyl)-fumaramid
- N-(5-Mercaptopentyl)-fumaramid
- N-[2-(2-Mercaptoethoxy)-ethyl]-fumaramid
- N-(2-Mercaptoethyl)-N'-methyl-fumaramid
- N-(2-Mercaptoethyl)-N',N'-dimethyl-fumaramid
- N-(2-Mercaptoethyl)-N'-ethyl-fumaramid
- N-(2-Mercaptoethyl)-N',N'-diethyl-fumaramid
- N-(2-Mercaptoethyl)-N'-(2-hydroxyethyl)-fumaramid
- N-(2-Mercaptoethyl)-N',N'-(2,2-dihydroxyethyl)-fumaramid
- N-(2-Mercaptoethyl)-N'-(3-dimethylaminopropyl)-fumaramid
- N-(2-Mercaptoethyl-terephthalamid
- N-(2-Mercaptoethyl)-N'-methyl-terephthalamid
- N-(2-Mercaptoethyl)-N'-ethyl-terephthalamid
- N-(2-Mercaptoethyl)-N'-(2-hydroxyethyl)-terephthalamid
- N-(2-Mercaptoethyl)-N'-(3-dimethylaminopropyl)-terephthalamid

**8.** Zusammensetzung nach den Ansprüchen 1 oder 2, dadurch gekennzeichnet, daß, wenn in der allgemeinen Formel (I) B die zweiwertige Gruppe $-(CH_2)_m-$ bedeutet, wobei m eine ganze Zahl im Bereich von 6 bis 7 ist, die Verbindungen ausgewählt sind unter:

- Octandisäure-1-amid-5-[(2-mercaptoethyl)-amid]
- Nonandisäure-1-amld-5-[(2-mercaptoethyl)-amid].

**9.** Zusammensetzung nach den Ansprüchen 1 oder 2, dadurch gekennzeichnet, daß, wenn in der allgemeinen Formel (I) B die zweiwertige Gruppe

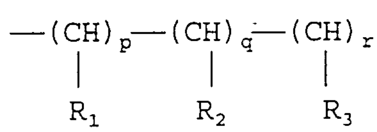

bedeutet, wobei $R_1$, $R_2$, $R_3$, p, q, r die für Anspruch 1 angegebenen Bedeutungen aufweisen, die Verbindungen ausgewählt sind unter:

- 3-Methyl-pentandisäure-1-amid-5-[(2-mercaptoethyl)-amid]
- 2-Ethyl-hexandisäure-1-amid-6-[(2-mercaptoethyl)-amid]

**10.** Zusammensetzung nach den Ansprüchen 1 oder 2, dadurch gekennzeichnet, daß, wenn in der allgemeinen Formel (I) B die zweiwertige Gruppe

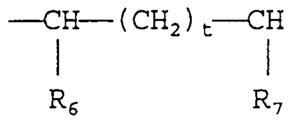

bedeutet, wobei $R_6$, $R_7$ und t die für Anspruch 1 angegebenen Bedeutungen aufweisen, die Verbindungen ausgewählt sind unter:

- 4-Amino-pentandisäure-1-amid-5-[(2-mercaptoethyl)-amid]
- 3-Amino-butandisäure-1-amid-5-[(2-mercaptoethyl)-amid].

**11.** Zusammensetzung nach einem der Ansprüche 1 bis 10, dadurch gekennzeichnet, daß die Verbindung der Formel (I) in einer Konzentration im Bereich von 0,5 bis 30 Gew.-% und vorzugsweise im Bereich von 5 bis 20 Gew.-%, bezogen auf das Gesamtgewicht der Zusammensetzung, vorliegt.

**12.** Zusammensetzung nach einem der Ansprüche 1 bis 11, dadurch gekennzeichnet, daß sie einen pH-Wert im Bereich von 4 bis 11 und vorzugsweise von 6 bis 10 aufweist, der mit einem Alkalisierungsmittel, das unter Ammoniak, Monoethanolamin, Diethanolamin, Triethanolamin, 1,3-Propandiamin, Ammoniumcarbamat, Alkalicarbonaten, Alkalihydrogencarbonaten, Ammoniumcarbonat, Ammoniumhydrogencarbonat, organischen Carbonaten oder Alkalihydroxiden ausgewählt ist, oder einem Mittel zum Ansäuern, das unter Salzsäure, Essigsäure, Milchsäure, Oxalsäure oder Borsäure ausgewählt ist, eingestellt wird.

**13.** Zusammensetzung nach einem der Ansprüche 1 bis 12, dadurch gekennzeichnet, daß sie ferner mindestens ein Disulfid enthält, wobei die Zusammensetzung vom selbstneutralisierenden Typ ist.

**14.** Zusammensetzung nach Anspruch 13, dadurch gekennzeichnet, daß das Disulfid der folgenden allgemeinen Formel entspricht:

$$-(-S-A-NH-CO-B-CO-NRR')_2 \qquad (II),$$

worin A, B, R und R' die für die allgemeine Formel (I) in Anspruch 1 angegebenen Bedeutungen aufweisen.

**15.** Zusammensetzung nach den Ansprüchen 13 oder 14, dadurch gekennzeichnet, daß das Disulfid in einem molaren Mengenanteil, bezogen auf die Verbindung der Formel (I), von 0,5 bis 2,5 und vorzugsweise 1 bis 2 vorliegt.

**16.** Verfahren zur dauerhaften Verformung des Haares, das darin besteht, in einem Schritt die Disulfidbindungen des Keratins durch Auftragen einer reduzierenden Zusammensetzung zu reduzieren und dann in einem zweiten Schritt die Bindungen durch Auftragen einer oxidierenden Zusammensetzung neu zu bilden, dadurch gekennzeichnet, daß der Reduktionsschritt mit einer kosmetischen reduzierenden Zusammensetzung nach einem der Ansprüche 1 bis 15 durchgeführt wird.

**17.** Verfahren zur Herstellung von Verbindungen der Formel (I) nach Anspruch 1, dadurch gekennzeichnet, daß es darin besteht, in einem ersten Schritt in einem inerten Lösungsmittel in Gegenwart eines Katalysators ein Aminothiol der Formel HS-A-$NH_2$ oder sein Hydrochlorid mit einem Dicarbonsäuremonoester der Formel (2)

$$HOOC-B-COOR_{12} \qquad (2)$$

umzusetzen, wobei A und B die für Anspruch 1 angegebenen Bedeutungen aufweisen und $R_{12}$ eine Alkylgruppe mit 1 bis 4 Kohlenstoffatomen ist, um den N-Mercaptoalkylester der Formel (3)

$$HS—A—NH—CO—B—COOR_{12} \quad (3)$$

herzustellen, der in einem zweiten Schritt mit einem Amin der Formel HNRR' gegebenenfalls in Gegenwart eines niederen Alkohols umgesetzt wird, wobei R und R' die für Anspruch 1 angegebenen Bedeutungen aufweisen.

**18.** Verfahren nach Anspruch 17, dadurch gekennzeichnet, daß das inerte Lösungsmittel im ersten Schritt unter Dichlormethan, 1,2-Dichlorethan, 1,1,1-Trichlorethan, Chloroform, Acetonitril, Toluol, Dioxan, Tetrahydrofuran, 1,2-Dimethoxyethan und Cyclohexan ausgewählt ist.

**19.** Verfahren nach einem der Ansprüche 17 bis 18, dadurch gekennzeichnet, daß im zweiten Schritt der Katalysator das Dicyclohexylcarbodiimid ist.

**20.** Neue Verbindungen, dadurch gekennzeichnet, daß sie der folgenden allgemeinen Formel (II) entsprechen:

$$—(—S—A—NH—CO—B—CO—NRR')_2 \quad (II),$$

worin bedeuten:

- A eine zweiwertige Gruppe $-(CH_2)_n-$, wobei n eine ganze Zahl im Bereich von 2 bis 5 ist, oder die zweiwertige Gruppe $-(CH_2)_2-O-(CH_2)_2-$,
- B eine Gruppe, die ausgewählt ist unter:

  (a) einer zweiwertigen Gruppe $-(CH_2)_m$, wobei m eine ganze Zahl im Bereich von 1 bis 7 ist,

  (b) einer zweiwertigen Gruppe

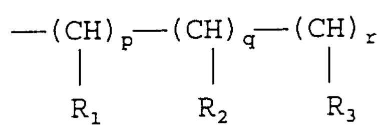

  worin eine der Gruppen $R_1$, $R_2$, $R_3$ unter den geradkettigen oder verzweigten Alkylgruppen mit 1 bis 4 Kohlenstoffatomen, Methoxymethyl, Phenyl, Benzyl, Cyclohexyl oder Cyclopentyl ausgewählt ist und die beiden anderen Gruppen Wasserstoff bedeuten, wobei p, q und r Null oder eine ganze Zahl im Bereich von 1 bis 4 bedeuten und ferner gilt: $1 \leq p + q + r \leq 4$,

  (c) einer zweiwertigen Gruppe

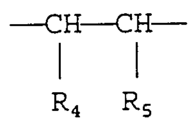

  worin $R_4$ oder $R_5$, die identisch oder voneinander verschieden sind, eine geradkettige oder verzweigte Alkylgruppe mit 1 bis 4 Kohlenstoffatomen bedeuten oder gemeinsam mit den angrenzenden Kohlenstoffatomen einen Cyclohexan- oder Cyclohexenring bilden,

  (d) einer zweiwertigen Grupp

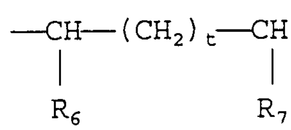

worin eine der Gruppen $R_6$ oder $R_7$ die Gruppe $-NH_2$ und die andere Gruppe ein Wasserstoffatom bedeutet, wobei t Null oder 1 ist, und

(e) die zweiwertige Gruppe

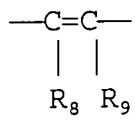

worin $R_8$ und $R_9$, die identisch oder voneinander verschieden sind, Wasserstoff oder eine geradkettige oder verzweigte Alkylgruppe mit 1 bis 4 Kohlenstoffatomen bedeuten oder gemeinsam mit den angrenzenden Kohlenstoffatomen einen Benzolring bilden.

- R und R', die identisch oder voneinander verschieden sind:

  . Wasserstoff oder eine Gruppe, die ausgewählt ist unter den geradkettigen oder verzweigten Alkylgruppen mit 1 bis 4 Kohlenstoffatomen und den geradkettigen oder verzweigten Hydroxyalkylgruppen mit 1 bis 5 Kohlenstoffatomen, oder

  . R bedeutet Wasserstoff und R' eine Aminoalkylgruppe $-(CH_2)_v-NR_{10}R_{11}$, worin v eine ganze Zahl von 1 bis 3 bedeutet und $R_{10}$ und $R_{11}$, die identisch oder voneinander verschieden sind, Wasserstoff oder eine geradkettige oder verzweigte Alkylgruppe mit 1 bis 3 Kohlenstoffatomen bedeuten, wobei $R_{10}$ und $R_{11}$ nicht gleichzeitig Wasserstoff bedeuten können,

und die organischen und anorganischen Salze der Verbindungen der Formel (II).

**21.** Neue Verbindungen, dadurch gekennzeichnet, daß sie der allgemeinen Formel (III) entsprechen:

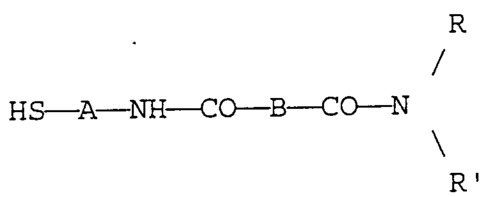

(I),

worin bedeuten:

- A eine zweiwertige Gruppe $-(CH_2)_n-$, wobei n eine ganze Zahl im Bereich von 2 bis 5 ist, oder die zweiwertige Gruppe $-(CH_2)_2-O-(CH_2)_2-$

- B eine Gruppe, die ausgewählt ist unter:

  (a) einer zweiwertigen Gruppe $-(CH_2)_m-$, wobei m eine ganze Zahl im Bereich von 1 bis 7 ist,

  (b) einer zweiwertigen Gruppe

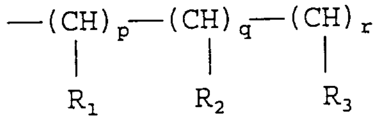

worin eine der Gruppen $R_1$, $R_2$, $R_3$ unter den geradkettigen oder verzweigten Alkylgruppen mit 1 bis 4

Kohlenstoffatomen, Methoxymethyl, Phenyl, Benzyl, Cyclohexyl oder Cyclopentyl ausgewählt ist und die beiden anderen Gruppen Wasserstoff bedeuten, wobei p, q und r Null oder eine ganze Zahl im Bereich von 1 bis 4 bedeuten und ferner gilt: $1 \leq p + q + r \leq 4$,

(c) einer zweiwertigen Gruppe

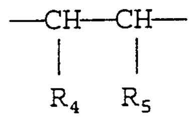

worin $R_4$ oder $R_5$, die identisch oder voneinander verschieden sind, eine geradkettige oder verzweigte Alkylgruppe mit 1 bis 4 Kohlenstoffatomen bedeuten oder gemeinsam mit den angrenzenden Kohlenstoffatomen einen Cyclohexan- oder Cyclohexenring bilden,

(d) einer zweiwertigen Gruppe

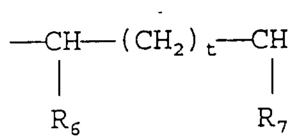

worin eine der Gruppen $R_6$ oder $R_7$ die Gruppe $-NH_2$ und die andere Gruppe ein Wasserstoffatom bedeutet, wobei t Null oder 1 ist, und

(e) einer zweiwertigen Gruppe

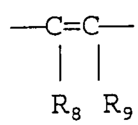

worin $R_8$ und $R_9$, die identisch oder voneinander verschieden sind, Wasserstoff oder eine geradkettige oder verzweigte Alkylgruppe mit 1 bis 4 Kohlenstoffatomen bedeuten oder gemeinsam mit den angrenzenden Kohlenstoffatomen einen Benzolring bilden.

- R und R', die identisch oder voneinander ver schieden sind:

  . entweder Wasserstoff oder eine Gruppe, die ausgewählt ist unter den geradkettigen oder verzweigten Alkylgruppen mit 1 bis 4 Kohlenstoffatomen und den geradkettigen oder verzweigten Hydroxyalkylgruppen mit 1 bis 5 Kohlenstoffatomen, oder

  . R bedeutet Wasserstoff und R' eine Aminoalkylgruppe $-(CH_2)_v-NR_{10}R_{11}$, worin v eine ganze Zahl von 1 bis 3 bedeutet und $R_{10}$ und $R_{11}$, die identisch oder voneinander verschieden sind, Wasserstoff oder eine geradkettige oder verzweigte Alkylgruppe mit 1 bis 3 Kohlenstoffatomen bedeuten, wobei $R_{10}$ und $R_{11}$ nicht gleichzeitig Wasserstoff sein können,

und die organischen und anorganischen Salze der Verbindungen der Formel (III), mit Ausnahme der folgenden Verbindungen:

(i) A bedeutet eine zweiwertige Gruppe $-(CH_2)_2-$ und B die Gruppe $-(CH_2)_m-$, wobei m eine Zahl im Bereich von 2 bis 7 ist,

oder die Gruppe

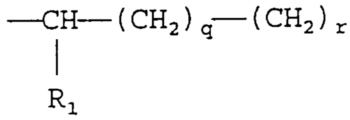

worin $R_1$ eine Gruppe bedeutet, die unter den geradkettigen oder verzweigten Alkylgruppen mit 1 bis 4 Kohlenstoffatomen, Phenyl, Benzyl, Cyclohexyl oder Cyclopentyl ausgewählt ist, $1 < 1 + q + r \leq 4$,
und R und R', die identisch oder voneinander verschieden sind bedeuten:

. Wasserstoff oder eine Gruppe, die unter den geradkettigen oder verzweigten Alkylgruppen mit 1 bis 4 Kohlenstoffatomen ausgewählt sind, oder

. R bedeutet Wasserstoff und R' eine Aminoalkylgruppe $-(CH_2)_v-NR_{10}R_{11}$, worin v eine ganze Zahl im Bereich von 1 bis 3 ist und $R_{10}$ und $R_{11}$, die identisch oder voneinander verschieden sind, Wasserstoff oder eine geradkettige oder verzweigte Alkylgruppe mit 1 bis 3 Kohlenstoffatomen bedeuten, wobei $R_{10}$ und $R_{11}$ nicht gleichzeitig Wasserstoff bedeuten können, oder

(ii) A bedeutet die zweiwertige Gruppe $-(CH_2)_2-$ und B die Gruppe $-CH_2-$ oder die Gruppe

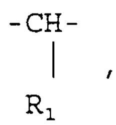

worin $R_1$ eine Gruppe bedeutet, die unter den geradkettigen oder verzweigten Alkylgruppen mit 1 bis 4 Kohlenstoffatomen, Methoxymethyl, Phenyl und Benzyl ausgewählt ist, und
R und R', die identisch oder voneinander verschieden sind, bedeuten:

. entweder Wasserstoff oder eine Gruppe, die unter den geradkettigen oder verzweigten Alkylgruppen mit 1 bis 4 Kohlenstoffatomen ausgewählt ist,

. oder R bedeutet Wasserstoff und R' eine Aminoalkylgruppe $-(CH_2)_v-NR_{10}R_{11}$, worin v eine ganze Zahl im Bereich von 1 bis 3 ist und $R_{10}$ und $R_{11}$, die identisch oder voneinander verschieden sind, eine geradkettige oder verzweigte Alkylgruppe mit 1 bis 3 Kohlenstoffatomen bedeuten.

**22.** Verbindungen nach Anspruch 21, dadurch gekennzeichnet, daß die Salze der Verbindungen der Formel (III) unter den Hydrochloriden, Hydrobromiden, Citraten, Oxalaten und Acetaten ausgewählt sind.

**23.** Verbindungen nach Anspruch 21, dadurch gekennzeichnet, daß, wenn in der allgemeinen Formel (III) B die zweiwertige Gruppe $-CH_2-$ bedeutet, die Verbindungen ausgewählt sind unter:

- N-(3-Mercaptopropyl)-malonamid
- N-(5-Mercaptopentyl)-malonamid
- N-[2-(2-Mercaptoethoxy)-ethyl]-malonamid
- N-(2-Mercaptoethyl)-N'-(2-hydroxyethyl)-malonamid
- N-(2-Mercaptoethyl)-N',N'-(2,2-dihydroxyethyl-malonamid.

**24.** Verbindungen nach Anspruch 21, dadurch gekennzeichnet, daß, wenn in der allgemeinen Formel (III) B die zweiwertige Gruppe $-(CH_2)_2-$ bedeutet, die Verbindungen ausgewählt sind unter:

- N-(3-Mercaptopropyl)-succinamid
- N-(5-Mercaptopentyl)-succinamid
- N-[2-(2-Mercaptoethoxy)-ethyl]-succinamid.

**25.** Verbindungen nach Anspruch 21, dadurch gekennzeichnet, daß, wenn in der allgemeinen Formel (III) B die zweiwertige Gruppe $-(CH_2)_3-$ bedeutet, die Verbindungen ausgewählt sind unter:

- N-(3-Mercaptopropyl)-glutaramid
- N-(5-Mercaptopentyl)-glutaramid
- N-[2-(2-Mercaptoethoxy)-ethyl]-glutaramid.

**26.** Verbindungen nach Anspruch 21, dadurch gekennzeichnet, daß, wenn in der allgemeinen Formel (III) B die zweiwertige Gruppe

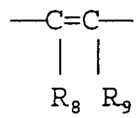

bedeutet, wobei $R_8$ und $R_9$ die für Anspruch 21 angegebenen Bedeutungen aufweisen, die Verbindungen ausgewählt sind unter:

- N-(2-Mercaptoethyl)-fumaramid
- N-(3-Mercaptopropyl)-fumaramid
- N-(5-Mercaptopentyl)-fumaramid
- N-[2-(2-Mercaptoethoxy)-ethyl]-fumaramid
- N-(2-Mercaptoethyl)-N'-methyl-fumaramid
- N-(2-Mercaptoethyl)-N',N'-dimethyl-fumaramid
- N-(2-Mercaptoethyl)-N'-ethyl-fumaramid
- N-(2-Mercaptoethyl)-N',N'-diethyl-fumaramid
- N-(2-Mercaptoethyl)-N'-(2-hydroxyethyl)-fumaramid
- N-(2-Mercaptoethyl)-N',N'-(2,2-dihydroxyethyl)-fumaramid
- N-(2-Mercaptoethyl)-N'-(3-dimethylaminopropyl)-fumaramid
- N-(2-Mercaptoethyl-terephthalamid
- N-(2-Mercaptoethyl)-N'-methyl-terephthalamid
- N-(2-Mercaptoethyl)-N'-ethyl-terephthalamid
- N-(2-Mercaptoethyl)-N'-(2-hydroxyethyl)-terephthalamid
- N-(2-Mercaptoethyl)-N'-(3-dimethylaminopropyl)-terephthalamid

**27.** Verbindungen nach Anspruch 21, dadurch gekennzeichnet, daß, wenn in der allgemeinen Formel (III) B die zweiwertige Gruppe

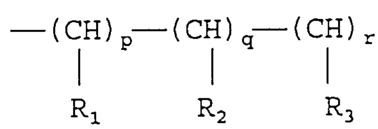

bedeutet, wobei $R_1$, $R_2$, $R_3$, p, q und r die für den Anspruch 21 angegebenen Bedeutungen aufweisen, die Verbindungen ausgewählt sind unter:

- 3-Methyl-pentandisäure-1-amid-5-[(2-mercaptoethyl)-amid]
- 2-Ethyl-hexandisäure-1-amid-6-[(2-mercaptoethyl)-amid].

**28.** Verbindungen nach Anspruch 21, dadurch gekennzeichnet, daß, wenn in der allgemeinen Formel (III) B die zweiwertige Gruppe

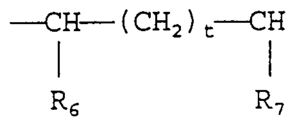

bedeutet, wobei $R_6$, $R_7$ und t die für die in Anspruch 21 angegebenen Bedeutungen aufweisen, die Verbindungen ausgewählt sind unter:

- 4-Amino-pentandisäure-1-amid-5-[(2-mercaptoethyl)-amid]
- 3-Amino-butandisäure-1-amid-5-[(2-mercaptoethyl)-amid].